(19) Europäisches Patentamt
European Patent Office
Office européen des brevets

(11) EP 2 439 200 A1

(12) **EUROPEAN PATENT APPLICATION**

(43) Date of publication:
**11.04.2012 Bulletin 2012/15**

(21) Application number: **10009835.9**

(22) Date of filing: **17.09.2010**

(51) Int Cl.:
***C07D 257/04*** (2006.01) ***C07C 309/53*** (2006.01)
***A61K 31/185*** (2006.01) ***A61P 1/04*** (2006.01)
***A61P 1/10*** (2006.01) ***A61P 9/00*** (2006.01)
***A61P 9/10*** (2006.01) ***A61P 11/06*** (2006.01)
***A61P 13/00*** (2006.01)

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Dundalk Institute of Technology**
**Co. Louth (IE)**

(72) Inventors:
- **Hollywood, Mark Anthony**
  **Saintfield, Country Down**
  **BT24 7WG, Northern Ireland (GB)**
- **Thornbury, Keith**
  **Banbridge**
  **BT32 3XY, Northern Ireland (GB)**
- **Sergeant, Gerard Patrick**
  **Belfast**
  **BT8 6WE, Northern Ireland (GB)**
- **McHale, Noel**
  **Belfast**
  **BT9 6NG, Northern Ireland (GB)**
- **Roy, Subhrangsu**
  **Garkamalpur, P.O. Mahishadal**
  **Purba-Midnapore**
  **721628 West Bengal (IN)**

(74) Representative: **Duffy, Assumpta Dympna et al**
**FRKelly**
**27 Clyde Road**
**Ballsbridge**
**Dublin 4 (IE)**

(54) **Anthraquinone compounds and their uses**

(57) This invention relates to compounds comprising a substituted or unsubstituted anthraquinone, or a salt or isomer thereof, for use in treating a disorder caused by or associated with dysfunctional ion channel activity; for example, BK Channel activity. The compounds of the present invention find utility in the treatment such as urinary incontinence, irritable bowel syndrome, diabetes and arterial hypertension, cardiovascular diseases including myocardial infarction, erectile dysfunction and airway constriction.

Figure 7

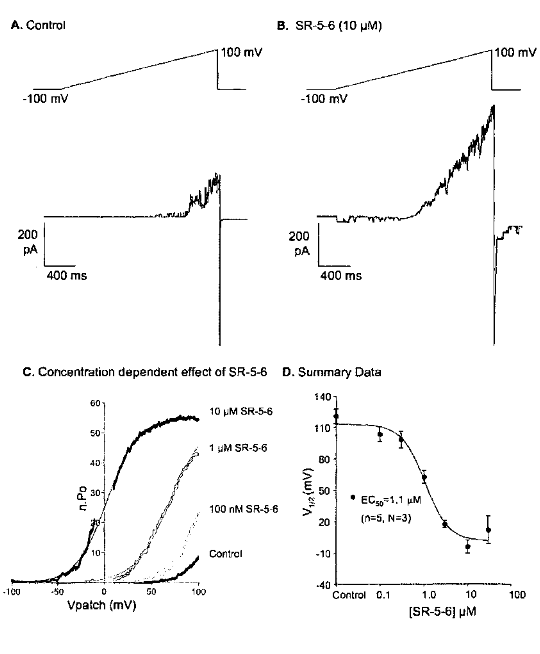


EP 2 439 200 A1

## Description

### Background to the Invention

Smooth muscle has been implicated to play a role in a large number of diseases affecting the urinary tract (e.g. urinary incontinence), the digestive system (e.g. irritable bowel syndrome), the circulatory system and the reproductive system. Large conductance potassium ion channels (BK channels), also called Maxi-K or slo1, are membrane associated ion channels, which conduct potassium ions across a cell membrane. BK channels are activated (opened) or deactivated (closed) by two physiologically relevant factors; a change in intracellular calcium ion ($Ca^{2+}$) concentration, or a change in the electrical potential across the cell membrane. An increase in the activity of BK channels leads to a decrease in cell excitability and a concurrent hyperpolarization of the cell membrane. As such, BK channels are critical in the regulation of smooth muscle tone, neuronal excitability, secretion, contractility to name a few.

Drugs that activate or open BK channels are in high demand for their potential clinical use. Primary indications for BK channel openers include urinary incontinence, irritable bowel syndrome, diabetes and arterial hypertension, cardiovascular diseases including myocardial infarction, erectile dysfunction and airway constriction.

Only one BK opener, Andolast (CR 2039, N-4-(5-tetrazolyl)-phenyl-4-(5-tetrazolyl)-benzamide) is currently in development, in phase 3 clinical trials in bronchial pneumonia patients in a comparison study with inhaled corticosteroids. Three other BK openers have failed in clinical development and have been discontinued. This class of drugs is however of exceptional importance and commercial value and has been of interest as a target for drug discovery, because experimental evidence suggests BK Channels play a pivotal and specific role in many pathophysiological conditions. This class of drug will elicit smooth muscle relaxation. As a result, in diseases such as urinary incontinence, where a hallmark of the disease or condition is overactive spastic smooth muscle, a BK channel opener will relax the spastic muscle, returning it to normal functioning and decreasing the urge that accompanies urinary incontinence.

The current market leader for the treatment of urinary incontinence is Detrol® (marketed by Pfizer), and all other drugs on the market used to treat this condition are of the same classification; muscarinic antagonists. Detrol® and similar muscarinic antagonists bind to, but do not activate muscarinic cholinergic receptors. Rather they act by blocking the action of endogenous acetylcholine, a neurotransmitter found in both peripheral and central nervous systems. These agents have widespread effects including actions on the iris and ciliary muscles in the eye and on organs such as the heart and vasculature, secretions associated with the respiratory tract, the GI system, salivary glands, and the CNS - contributing to a plethora of side effects from the drugs. The most common side effects from muscarinic antagonists like Detrol® include blurred vision, constipation, dizziness, drowsiness, dry eyes, dry mouth, headache, indigestion, stomach pain. More severe side effects can include severe allergic reactions such as a rash, hives, itching, difficulty breathing, tightness in the chest, swelling of the mouth, face, lips, or tongue, unusual hoarseness, chest pain, confusion, difficult or painful urination, disorientation, fast or irregular heart beat, hallucinations, memory problems, severe dizziness, swelling of the hands, ankles or feet.

Approximately one in 6 people in the USA are affected by overactive bladder. The condition is the result of bladder muscle contraction and squeezing too often, causing frequent and strong urges to urinate, in addition to undesired wetting incidences affecting sleep, social life, health and well-being, relationships and feelings of self-worth.

### Summary of the Invention

According to a first aspect of the present invention, there is provided a compound comprising a substituted or unsubstituted anthraquinone, or a salt or isomer thereof, for use in treating a disorder caused by or associated with dysfunctional ion channel activity.

Optionally, the compound has the general formula (I):

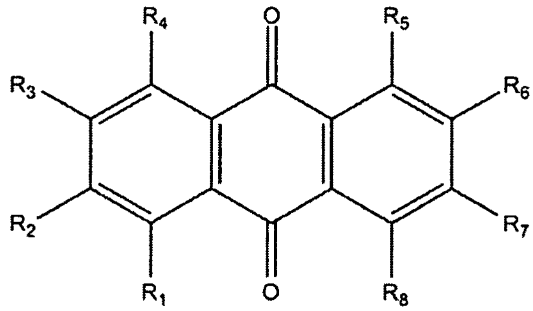

(I)

wherein;
$R_1$-$R_4$ and $R_7$ are each a hydrogen atom;
$R_5$ and $R_8$, which can be the same or different, are each independently selected from a hydrogen atom; and an amine, optionally a secondary amine; and $R_6$ is independently selected from a hydrogen atom or a alkyl group; further optionally a sulfonate or a carboxyl group.

Optionally, the compound comprises a substituted anthraquinone, or a salt or isomer thereof.

Optionally, the compound comprises an acid dye, or a salt or isomer thereof, which can be substituted or unsubstituted. Further optionally, the acid dye, or a salt or isomer thereof, is substituted.

Optionally, the compound comprises an acid dye selected from an acid blue dye and an acid green dye, or a salt or isomer each thereof, which can be substituted or unsubstituted.

Optionally, the compound comprises a substituted anthraquinone and has the general formula (IA):

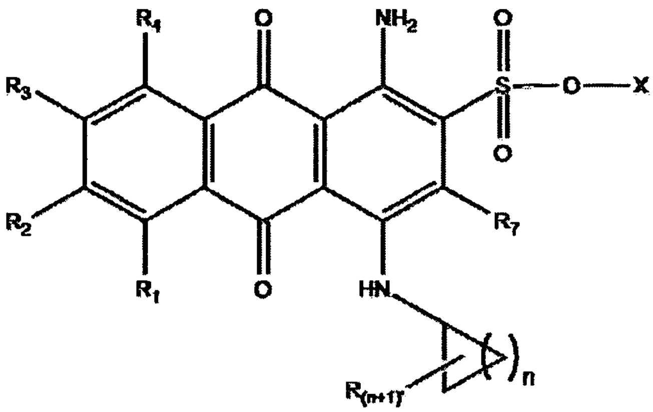

(IA)

wherein;
n is an integer from 1 to 5;
$R_1$-$R_4$ and $R_7$ are each a hydrogen atom;
X is an alkali metal cation, optionally sodium;
each $R_{(n+1)'}$ is independently selected from at least one of:

i. a hydrogen atom;
ii. a heteroatom selected from a halide, optionally fluoride or chloride; an oxygen atom; or an amine, optionally a primary amine;
iii. a short chain alkyl, alkenyl, or alkynyl group, which can be branched or unbranched, substituted or unsubstituted, linear or cyclic; optionally a short chain alkyl; further optionally an ethyl, propyl, or a butyl group;
iv. a short chain aza-alkyl, aza-alkenyl, or aza-alkynyl group, which can be branched or unbranched, substituted or unsubstituted, linear or cyclic;
v. a short chain alkoxyl, alkenoxyl, or alkynoxyl group, which can be branched or unbranched, substituted or unsubstituted, linear or cyclic;

vi. a short chain thio-alkyl, thio-alkenyl, or thio-alkynyl group, which can be branched or unbranched, substituted or unsubstituted, linear or cyclic;
vii. a short chain halo-alkyl, halo-alkenyl, or halo-alkynyl group, which can be branched or unbranched, substituted or unsubstituted, linear or cyclic; optionally a fluorinated methyl group; further optionally a trifluoromethyl group;
viii. a nitrile, hydroxyl or ester group;
ix. a sulfonate or carboxyl group; and
x. a tetrazole.

or a pharmaceutically acceptable salt, hydrate, or isomer thereof.

By the term "linear" is meant a molecule comprising at least two atoms, any of which can be the same or different, wherein each atom of the molecule is bonded to an adjacent atom in a substantially straight series. Each atom can be bonded to an adjacent carbon atom by a single-, double-, triple-, or higher order-bond.

By the term "cyclic" is meant a molecule comprising at least three atoms, any of which can be the same or different, wherein each atom of the molecule is bonded to an adjacent atom in a substantially continuous series having no terminal atoms. Each atom can be bonded to an adjacent carbon atom by a single-, double-, triple-, or higher order-bond.

By the term "branched" is meant a molecule comprising at least three atoms, any of which can be the same or different, bonded in a substantially straight series, wherein the molecule further comprises at least one other atom, which is not bonded to either of the terminal atoms of the substantially straight series. Each atom can be bonded to an adjacent atom by a single-, double-, triple-, or higher order-bond.

By "short chain" is meant a polyatomic molecule comprising at least one carbon atom. Optionally, the polyatomic molecule comprises 1-6 carbon atoms. Further optionally, the polyatomic molecule comprises 1-3 carbon atoms. Optionally, the alkyl group comprises two carbon atoms. Optionally, the alkyl group is an ethyl group.

Alternatively, the alkyl group comprises three carbon atoms. Optionally, the alkyl group is a propyl group, optionally an isopropyl group.

Further alternatively, the alkyl group comprises four carbon atoms. Optionally, the alkyl group is a butyl group, optionally a tertiary butyl group.

Optionally, the alkenyl group comprises at least six carbon atoms. Further optionally, the alkenyl group is a cyclic, for example a phenyl, group.

Optionally, the short chain alkyl, alkenyl, or alkynyl group is a polycyclic group.

Optionally, the polycyclic group is a cycloalkane, cycloalkene, or cycloalkyne. Still further optionally, the polycyclic group comprises a cycloalkene, for example a phenyl, group; and a cycloalkane fused, optionally ortho-fused, thereto. Optionally or additionally, the polycyclic group comprises a cycloalkene, for example a phenyl, group; and a cycloalkane fused , optionally ortho-fused, thereto, wherein the cycloalkane comprises 1 to 6 carbon atoms; optionally 3 to 4 carbon atoms.

Optionally or additionally, the polycyclic group comprises a cycloalkene, for example a phenyl, group; and a cycloalkane fused, optionally ortho-fused, thereto, wherein the cycloalkane comprises cyclopentane.

Optionally or additionally, the polycyclic group comprises a cycloalkene, for example a phenyl, group; and a cycloalkane fused, optionally ortho-fused, thereto, wherein the cycloalkane comprises cyclohexane.

Optionally, the polycyclic group comprises a cycloalkene, for example a phenyl, group; and a cycloalkene fused, optionally ortho-fused, thereto. Optionally or additionally, the polycyclic group comprises a cycloalkene, for example a phenyl group; and a cycloalkene fused, optionally ortho-fused, thereto, wherein each cycloalkene comprises 1 to 6 carbon atoms.

Optionally, the polycyclic group comprises naphthalene.

Optionally, the halo-alkyl group comprises at least one carbon atom and at least one fluorine atom. Optionally, the haloalkyl group is a trifluoromethyl group. Optionally, the haloalkyl group comprises a trifluoromethyl group bonded to at least one heteroatom. Further optionally, the haloalkyl group comprises a trifluoromethyl group bonded to at least one oxygen atom ($-O-CF_3$).

Optionally, the alkoxyl group comprises at least one carbon atom and at least one oxygen atom. Optionally, the alkoxyl group is a methoxyl group.

Optionally, the alkenoxyl group is a carboxyl group.

Optionally, n is selected from 1, 3, 4, and 5.

Optionally, n is 1. Further optionally or additionally, each $R_{(n+1)'}$ is a hydrogen atom. Further optionally, the compound is sodium 1-amino-4-(cyclopropylamino)-9,10-dioxo-9,10-dihydroanthracene-2-sulfonate.

Optionally, n is 3. Further optionally or additionally, each $R_{(n+1)'}$ is a hydrogen atom. Further optionally, the compound is sodium 1-amino-4-(cyclopentylamino)-9,10-dioxo-9,10-dihydroanthracene-2-sulfonate.

Optionally, n is 4. Further optionally or additionally, each $R_{(n+1)'}$ is a hydrogen atom. Further optionally, the compound is sodium 1-amino-4-(cyclohexylamino)- 9,10-dioxo-9,10-dihydroanthracene-2-sulfonate.

Optionally, n is 4, and forms a cyclohexane. Further optionally or additionally, at least one $R_{(n+1)'}$ is a trifluoromethyl group. Still further optionally, $R(_{n+1)'}$ is a trifluoromethyl group attached at the 3-position of the cyclohexane. Further optionally, the compound is sodium 1-amino-9,10-dioxo-4-(3-(trifluoromethyl)cyclohexylamino)-9,10-dihydroanthracene-2-sulfonate.

Optionally, n is 4, and forms a cyclohexane. Further optionally or additionally, at least one $R_{(n+1)'}$ is an alkyl, optionally a butyl, group. Still further optionally, at least one $R_{(n+1)'}$ is a alkyl, optionally a tertiary butyl group; optionally attached at the 4-position of the cyclohexane. Further optionally, the compound is sodium 1-amino-4-(4-tert-butylcyclohexylamino)-9,10-dioxo-9,10-dihydroanthracene-2-sulfonate.

Optionally, n is 5. Further optionally or additionally, each $R_{(n+1)'}$ is a hydrogen atom. Further optionally, the compound is sodium 1-amino-4-(cycloheptylamino)-9,10-dioxo-9,10-dihydroanthracene-2-sulfonate.

Optionally, n is 4. Further optionally, the compound has the general formula (IB):

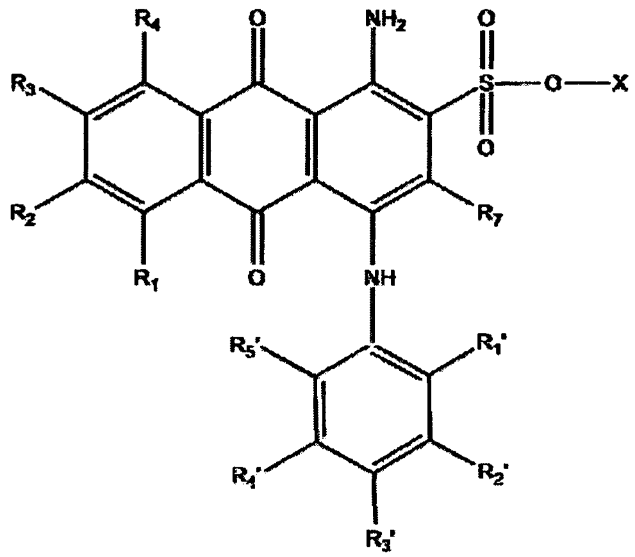

(IB)

Optionally, $R_1$ and $R_{5'}$, which can be the same or different, are each independently selected from at least one of each of:

i. a hydrogen atom;
ii. a heteroatom selected from a halide, optionally fluoride or chloride; or an oxygen atom;
iii. a short chain alkyl, alkenyl, or alkynyl group, which can be branched or unbranched, substituted or unsubstituted, linear or cyclic; optionally a short chain alkyl; further optionally an ethyl group;
iv. a short chain alkoxyl, alkenoxyl, or alkynoxyl group, which can be branched or unbranched, substituted or unsubstituted, linear or cyclic; and
v. a short chain halo-alkyl, halo-alkenyl, or halo-alkynyl group, which can be branched or unbranched, substituted or unsubstituted, linear or cyclic.

Further optionally, $R_1$ and $R_{5'}$, which can be the same or different, are each independently selected from at least one of each of:

i. a hydrogen atom;
ii. fluoride;
iii. a polycyclic group selected from a cycloalkane, cycloalkene, or cycloalkyne;
iv. a methoxyl group; and
v. a trifluoromethyl group.

Optionally, $R_{2'}$ and $\mathbf{R_{4'}}$, which can be the same or different, are each independently selected from at least one of each of:

i. a hydrogen atom;
ii. a heteroatom selected from a halide, optionally fluoride or chloride; an oxygen atom; or an amine, optionally a primary amine;
iii. a short chain halo-alkyl, halo-alkenyl, or halo-alkynyl group, which can be branched or unbranched, substituted or unsubstituted, linear or cyclic;
iv. a sulfonate or carboxyl group;
v. a short chain alkyl, alkenyl, or alkynyl group, which can be branched or unbranched, substituted or unsubstituted, linear or cyclic;
vi. a short chain alkoxyl, alkenoxyl, or alkynoxyl group, which can be branched or unbranched, substituted or unsubstituted, linear or cyclic;
vii. a nitrile group; and
viii. a tetrazole.

Further optionally, $R_{2'}$ and $R_{4'}$, which can be the same or different, are each independently selected from at least one of each of:

i. a hydrogen atom;
ii. fluoride;
iii. chloride;
iv. a trifluoromethyl group;
v. a trifluoromethyl group bonded to at least one oxygen atom ($-O-CF_3$);
vi. a methyl group;
vii. an ethyl group;
viii. an isopropyl group;
ix. a tert-butyl group;
x. a cyclopropyl group;
xi. a nitrile group;
xii. a methoxyl group;
xiii. a ethoxyl group;
xiv. an isopropoxyl group;
xv. an amine, optionally a primary amine;
xvi. a polycyclic group selected from a cycloalkane, cycloalkene, or cycloalkyne;
xvii. a benzyl group; and
xviii. a tetrazole.

Optionally, $R_{3'}$ is independently selected from at least one of each of:

i. a hydrogen atom;
ii. a short chain halo-alkyl, halo-alkenyl, or halo-alkynyl group, which can be branched or unbranched, substituted or unsubstituted, linear or cyclic;
iii. a heteroatom selected from a halide, optionally fluoride or chloride; an oxygen atom; or an amine, optionally a primary amine;
iv. a short chain alkyl, alkenyl, or alkynyl group, which can be branched or unbranched, substituted or unsubstituted, linear or cyclic
v. a short chain alkoxyl, alkenoxyl, or alkynoxyl group, which can be branched or unbranched, substituted or unsubstituted, linear or cyclic; and
vi. a nitrile.

Further optionally, $R_{3'}$. is independently selected from at least one of each of:

i. a hydrogen atom;
ii. a trifluoromethyl group;
iii. fluoride;
iv. chloride;
v. a benzyl group;
vi. a methyl group;
vii. a methoxyl group;
viii. an amine, optionally a primary amine; and

ix. a nitrile.

Optionally, each of $R_{1'}$-$R_{5'}$ is a hydrogen atom. Further optionally, the compound is sodium 1-amino-9,10-dioxo-4-(phenylamino)-9,10-dihydroanthracene-2-sulfonate.

Optionally, $R_{2'}$ is a trifluoromethyl group. Further optionally or additionally, each of $R_{1'}$, and $R_{3'}$,-$R_{5'}$ is a hydrogen atom. Further optionally, the compound is sodium 1-amino-9,10-dioxo-4-(3-(trifluoromethyl)phenylamino)-9,10-dihydroanthracene-2-sulfonate.

Optionally, $R_{1'}$ is a fluorine atom. Further optionally or additionally, $R_{5'}$ is a fluorine atom. Further optionally or additionally, each of $R_{2'}$-$R_{4'}$ is a hydrogen atom. Further optionally, the compound is sodium 1-amino-4-(2,6-difluorophenylamino)-9,10-dioxo-9,10-dihydroanthracene-2-sulfonate.

Optionally, $R_{1'}$ is a methoxyl group. Further optionally or additionally, each of $R_{2'}$-$R_{4'}$ is a hydrogen atom. Further optionally, the compound is sodium 1-amino-4-(2-methoxyphenylamino)-9,10-dioxo-9,10-dihydroanthracene-2-sulfonate.

Optionally, $R_{2'}$ is a trifluoromethyl group. Further optionally or additionally, $R_{4'}$ is a trifluoromethyl group. Further optionally or additionally, each of $R_{1'}$, $R_{3'}$, and $R_{5'}$ is a hydrogen atom. Further optionally, the compound is sodium 4-(3,5-bis(trifluoromethyl)phenylamino)-1-amino-9,10-dioxo-9,10-dihydroanthracene-2-sulfonate.

Optionally, $R_{2'}$ is fluoride. Further optionally or additionally, $R_{4'}$ is fluoride. Further optionally or additionally, each of $R_{1'}$, $R_{3'}$, and $R_{5'}$ is a hydrogen atom. Further optionally, the compound is sodium 1-amino-4-(3,5-difluorophenylamino)-9,10-dioxo-9,10-dihydroanthracene-2-sulfonate.

Optionally, $R_{2'}$ is fluoride. Further optionally or additionally, each of $R_{1'}$ and $R_{3'}$-$R_{5'}$ is a hydrogen atom. Further optionally, the compound is sodium 1-amino-4-(3-fluorophenylamino)-9,10-dioxo-9,10-dihydroanthracene-2-sulfonate.

Optionally, $R_{2'}$ is a sulfonic acid group. Further optionally or additionally, each of $R_{1'}$ and $R_{3'}$-$R_{5'}$ is a hydrogen atom. Further optionally, the compound is sodium 1-amino-9,10-dioxo-4-(3-sulfophenylamino)-9,10-dihydroanthracene-2-sulfonate.

Optionally, $R_{2'}$ is a trifluoromethyl group. Further optionally or additionally, $R_{4'}$ is fluoride. Further optionally or additionally, each of $R_{1'}$, $R_{3'}$, and $R_{5'}$ is a hydrogen atom. Further optionally, the compound is sodium 1-amino-4-(3-fluoro-5-(trifluoromethyl)phenylamino)-9,10-dioxo-9,10-dihydroanthracene-2-sulfonate.

Optionally, $R_{5'}$ is a trifluoromethyl group. Further optionally or additionally, each of $R_{1'}$-$R_{4'}$ is a hydrogen atom. Further optionally, the compound is sodium 1-amino-9,10-dioxo-4-(2-(trifluoromethyl)phenylamino)-9,10-dihydroanthracene-2-sulfonate.

Optionally, $R_{2'}$ is a methyl group. Further optionally or additionally, each of $R_{1'}$ and $R_{3'}$-$R_{5'}$ is a hydrogen atom. Further optionally, the compound is sodium 4-(m-toluidino)-1-amino-9,10-dioxo-9,10-dihydroanthracene-2-sulfonate.

Optionally, $R_{2'}$ is fluoride. Further optionally or additionally, $R_{3'}$ is a trifluoromethyl group. Further optionally or additionally, each of $R_{1'}$, $R_{4'}$, and $R_{5'}$ is a hydrogen atom. Further optionally, the compound is sodium 1-amino-4-(3-fluoro-4-(trifluoromethyl)phenylamino)-9,10-dioxo-9,10-dihydroanthracene-2-sulfonate.

Optionally, $R_{2'}$ is a trifluoromethyl group. Further optionally or additionally, $R_{3'}$ is fluoride. Further optionally or additionally, each of $R_{1'}$, $R_{4'}$, and $R_{5'}$ is a hydrogen atom. Further optionally, the compound is sodium 1-amino-4-(4-fluoro-3-(trifluoromethyl)phenylamino)-9,10-dioxo-9,10-dihydroanthracene-2-sulfonate.

Optionally, $R_{3'}$ is a trifluoromethyl group. Further optionally or additionally, each of $R_{1'}$, $R_{2'}$, $R_{4'}$, and $R_{5'}$ is a hydrogen atom. Further optionally, the compound is sodium 1-amino-9,10-dioxo-4-(4-(trifluoromethyl)phenylamino)-9,10-dihydroanthracene-2-sulfonate.

Optionally, $R_{2'}$ is an ethyl group. Further optionally or additionally, each of $R_{1'}$ and $R_{3'}$-$R_{5'}$ is a hydrogen atom. Further optionally, the compound is sodium 1-amino-4-(3-ethylphenylamino)-9,10-dioxo-9,10-dihydroanthracene-2-sulfonate.

Optionally, $R_{2'}$ is a trifluoromethoxyl group ($-OCF_3$). Further optionally or additionally, each of $R_{1'}$ and $R_{3'}$-$R_{5'}$ is a hydrogen atom. Further optionally, the compound is sodium 1-amino-9,10-dioxo-4-(3-(trifluoromethoxy)phenylamino)-9,10-dihydroanthracene-2-sulfonate.

Optionally, $R_{3'}$ is a benzyl group. Further optionally or additionally, each of $R_{1}$, $R_{2'}$, $R_{4'}$, and $R_{5'}$ is a hydrogen atom. Further optionally, the compound is sodium 1-amino-4-(4-benzylphenylamino)-9,10-dioxo-9,10-dihydroanthracene-2-sulfonate.

Optionally, $R_{2'}$ is a trifluoromethyl group. Further optionally or additionally, $R_{3'}$ is chloride. Further optionally or additionally, each of $R_{1'}$, $R_{4'}$, and $R_{5'}$ is a hydrogen atom. Further optionally, the compound is sodium 1-amino-4-(4-chloro-3-(trifluoromethyl)phenylamino)-9,10-dioxo-9,10-dihydroanthracene-2-sulfonate.

Optionally, $R_{2'}$ is trifluoromethyl group. Further optionally or additionally, $R_{3'}$ is a methyl group. Further optionally or additionally, each of $R_{1'}$, $R_{4'}$, and $R_{5'}$ is a hydrogen atom. Further optionally, the compound is sodium 1-amino-4-(4-methyl-3-(trifluoromethyl)phenylamino)-9,10-dioxo-9,10-dihydroanthracene-2-sulfonate.

Optionally, $R_{2'}$ is chloride. Further optionally or additionally, each of $R_{1'}$, and $R_{3'}$-$R_{5'}$ is a hydrogen atom. Further optionally, the compound is sodium 1-amino-4-(3-chlorophenylamino)-9,10-dioxo-9,10-dihydroanthracene-2-sulfonate.

Optionally, $R_{2'}$ is a nitrile group. Further optionally or additionally, each of $R_1$, and $R_{3'}$-$R_{5'}$ is a hydrogen atom. Further optionally, the compound is sodium 1-amino-4-(3-cyanophenylamino)-9,10-dioxo-9,10-dihydroanthracene-2-sulfonate.

Optionally, $R_{2'}$ is a tetrazole. Further optionally or additionally, each of $R_{1'}$, and $R_{3'}$-$R_{5'}$ is a hydrogen atom. Further optionally, the compound is sodium 4-(3-(1 H-tetrazol-5-yl)phenylamino)-1-amino-9,10-dioxo-9,10-dihydroanthracene-2-sulfonate.

Optionally, $R_{2'}$ is a trifluoromethyl group. Further optionally or additionally, $R_{3'}$ is a nitrile group. Further optionally or additionally, each of $R_1$, $R_{4'}$, and $R_{5'}$ is a hydrogen atom. Further optionally, the compound is sodium 1-amino-4-(4-cyano-3-(trifluoromethyl)phenylamino)-9,10-dioxo-9,10-dihydroanthracene-2-sulfonate.

Optionally, $R_{2'}$ is a trifluoromethyl group. Further optionally or additionally, $R_{3'}$ is a methoxyl group. Further optionally or additionally, each of $R_{1'}$, $R_{4'}$, and $R_{5'}$ is a hydrogen atom. Further optionally, the compound is sodium 1-amino-4-(4-methoxy-3-(trifluoromethyl)phenylamino)-9,10-dioxo-9,10-dihydroanthracene-2-sulfonate.

Optionally, $R_{2'}$ is a methoxyl group. Further optionally or additionally, $R_{4'}$ is a trifluoromethyl group. Further optionally or additionally, each of $R_{1'}$, $R_{3'}$, and $R_{5'}$ is a hydrogen atom. Further optionally, the compound is sodium 1-amino-4-(3-methoxy-5-(trifluoromethyl)phenylamino)-9,10-dioxo-9,10-dihydroanthracene-2-sulfonate.

Optionally, $R_{2'}$ is a trifluoromethyl group. Further optionally or additionally, $R_{3'}$ is an amine, optionally a primary amine. Further optionally or additionally, each of $R_{1'}$, $R_{4'}$, and $R_{5'}$ is a hydrogen atom. Further optionally, the compound is sodium 1-amino-4-(4-amino-3-(trifluoromethyl)phenylamino)-9,1 0-dioxo-9,1 0-dihydroanthracene-2-sulfonate.

Optionally, $R_{2'}$ is an amine, further optionally a primary amine. Optionally or additionally, $R_{4'}$ is a trifluoromethyl group. Further optionally or additionally, each of $R_{1'}$, $R_{3'}$, and $R_{5'}$ is a hydrogen atom. Further optionally, the compound is sodium 1-amino-4-(3-amino-5-(trifluoromethyl)phenylamino)-9,10-dioxo-9,10-dihydroanthracene-2-sulfonate.

Optionally, a cycloalkane is fused, optionally ortho-fused, at $R_{4'}$ and $R_{5'}$, wherein the cycloalkane comprises cyclohexane. Further optionally or additionally, each of $R_{1'}$-$R_{3'}$ is a hydrogen atom. Further optionally, the compound is sodium 1-amino-9,10-dioxo-4-(5,6,7,8-tetrahydronaphthalen-1-ylamino)-9,10-dihydroanthracene-2-sulfonate.

Optionally, a cycloalkane is fused, optionally ortho-fused, at $R_{2'}$ and $R_{3'}$, wherein the cycloalkane comprises cyclopentane. Further optionally or additionally, each of $R_{1'}$, $R_{4'}$, $R_{5'}$ is a hydrogen atom. Further optionally, the compound is sodium 1-amino-4-(2,3-dihydro-1H-inden-5-ylamino)-9,10-dioxo-9,10-dihydroanthracene-2-sulfonate.

Optionally, a cycloalkene is fused, optionally ortho-fused, at $R_{4'}$ and $R_{5'}$, wherein the cycloalkene comprises benzene. Further optionally or additionally, each of $R_{1'}$-$R_{3'}$ is a hydrogen atom. Further optionally, the compound is sodium 1-amino-4-(naphthalen-1-ylamino)-9,10-dioxo-9,10-dihydroanthracene-2-sulfonate.

Optionally, $R_{2'}$ is a benzyl group. Further optionally, or additionally, each of $R_{1'}$ and $R_{3'}$-$R_{5'}$ is a hydrogen atom. Further optionally, the compound is sodium 1-amino-4-(3-benzylphenylamino)-9,10-dioxo-9,10-dihydroanthracene-2-sulfonate.

Optionally, a cycloalkene is fused, optionally ortho-fused, at $R_{2'}$ and $R_{3'}$, wherein the cycloalkene comprises benzene. Further optionally or additionally, each of $R_{1'}$, $R_{4'}$, $R_{5'}$ is a hydrogen atom. Further optionally, the compound is sodium 1-amino-4-(naphthalen-2-ylamino)-9,10-dioxo-9,10-dihydroanthracene-2-sulfonate.

Optionally, a cycloalkane is fused, optionally ortho-fused, at $R_{2'}$ and $R_{3'}$, wherein the cycloalkane comprises cyclohexane. Further optionally or additionally, each of $R_{1'}$, $R_{4'}$, $R_{5'}$ is a hydrogen atom. Further optionally, the compound is sodium 1-amino-9,10-dioxo-4-(5,6,7,8-tetrahydronaphthalen-2-ylamino)-9,10-dihydroanthracene-2-sulfonate.

Optionally, $R_{2'}$ is a cyclopropyl group. Further optionally or additionally, each of $R_{1'}$ and $R_{3'}$-$R_{5'}$ is a hydrogen atom. Further optionally, the compound is sodium 1-amino-4-(3-cyclopropylphenylamino)-9,10-dioxo-9,10-dihyd roanthracene-2-sulfonate.

Optionally, $R_{2'}$ is an isopropyl group. Further optionally or additionally, each of $R_{1'}$ and $R_{3'}$-$R_{5'}$ is a hydrogen atom. Further optionally, the compound is sodium 1-amino-4-(3-isopropylphenylamino)-9,10-dioxo-9,10-dihydroanthracene-2-sulfonate.

Optionally, $R_{2'}$ is a tert-butyl group. Further optionally or additionally, each of $R_{1'}$ and $R_{3'}$-$R_{5'}$ is a hydrogen atom. Further optionally, the compound is sodium 1-amino-4-(3-tert-butylphenylamino)-9,10-dioxo-9,10-dihydroanthracene-2-sulfonate.

Optionally, $R_{2'}$ is an ethoxyl group. Further optionally or additionally, each of $R_{1'}$ and $R_{3'}$-$R_{5'}$ is a hydrogen atom. Further optionally, the compound is sodium 1-amino-4-(3-ethoxyphenylamino)-9,10-dioxo-9,10-dihydroanthracene-2-sulfonate.

Optionally, $R_{2'}$ is an isopropoxyl group. Further optionally or additionally, each of $R_{1'}$ and $R_{3'}$-$R_{5'}$ is a hydrogen atom. Further optionally, the compound is sodium 1-amino-4-(3-isopropoxyphenylamino)-9,10-dioxo-9,10-dihydroanthracene-2-sulfonate.

Optionally, $R_{1'}$ is a methoxyl group. Further optionally or additionally, $R_{4'}$ is a trifluoromethyl group. Further optionally or additionally, each of $R_{2'}$, $R_{3'}$, and $R_{5'}$ is a hydrogen atom. Further optionally, the compound is sodium 1-amino-4-(2-methoxy-5-(trifluoromethyl)phenylamino)-9,10-dioxo-9,10-dihydroanthracene-2-sulfonate.

By "dysfunctional" is meant any disturbance resulting in the abnormal functioning of a process, whereby the

process no longer follows a conventional functional pattern. The abnormal functioning of the process involves: impaired ion channel activity, the treatment of which comprises enhancement of ion channel activity; and enhanced ion channel activity, the treatment comprising impairment of ion channel activity.

By "impaired ion channel activity" is meant reduced capability of the ion channel to allow passage of ions there through. By "enhancement of ion channel activity" is meant increasing the capability of the ion channel to allow passage of ions there through.

Optionally, the ion channel is a potassium ion channel. Further optionally, the ion channel is a calcium-activated potassium ion channel. Still further optionally, the ion channel is a BK Channel.

Optionally, the disorder is a disorder associated with smooth muscle tone and contraction.

Optionally, the disorder is a disorder associated with smooth muscle tone and contraction in the circulatory system. Further optionally, the disorder is arterial hypertension including decreased blood flow or increased blood pressure. Still further optionally, the disorder is a cardiovascular disorder, optionally myocardial infarction.

Optionally, the disorder is a disorder associated with smooth muscle tone and contraction in the digestive system. Further optionally, the disorder contributes to or manifests as irritable bowel syndrome including incontinence, optionally faecal incontinence; constipation; gastrooesophageal reflux; and impaired gastrointestinal passage.

Optionally, the disorder is associated with smooth muscle tone and contraction in the genitourinary system. Further optionally, the disorder is incontinence, optionally urinary incontinence. Still further optionally, the disorder is erectile dysfunction.

Optionally, the disorder is associated with smooth muscle tone and contraction in the respiratory system. Further optionally, the disorder is associated with constriction of the airway. Still further optionally, the disorder is asthma.

Examples

All experiments were carried out at 36 ± 1 °C, and for the excised patch single channel recordings, the total **Ca**$^{2+}$ concentration required to give the free $Ca^{2+}$ stated in the text was calculated using Chelator software: (http://www.organphy.science.ru.nl/chelator/Chelmain.html)

All experiments were approved by the Dundalk Institute of Technology Animal Care and Use Committee. Tissues were obtained from male and female New Zealand white rabbits immediately after they had been killed by lethal injection of pentobarbitone. The urinary bladder and most proximal 1.5 cm of the urethra was removed and placed in Krebs solution. Strips of bladder tissue, 0.5 cm in width were dissected, cut into 1 $mm^3$ pieces and stored in $Ca^{2+}$-free Hanks' solution for 30 min prior to cell dispersal. Tissue pieces were incubated in dispersal medium containing (per 5 ml) of $Ca^{2+}$-free Hanks' solution: 15 mg collagenase (Sigma type 1A), 1 mg protease (Sigma type XXIV), 10 mg bovine serum albumin (Sigma) and 10 mg trypsin inhibitor (Sigma) for 10-15 min at 37°C. Tissue was then transferred to $Ca^{2+}$-free Hanks' solution and stirred for a further 10-15 min to release single smooth muscle cells. These were plated in Petri dishes containing 100 μM $Ca^{2+}$ Hanks' solution and stored at 4°C for use within 8 h. During experiments, the dish containing the cells was continuously perfused with Hanks' solution at 36 ± 1°C. Additionally the cell under study was continuously superfused by means of a custom built close delivery system with a pipette of tip diameter 200 μm placed approximately 300 μm from the cell. The high $K^+$ solution in the close delivery system could be switched to a drug-containing solution with a dead space time of less than 5 s.

For whole cell recordings pipettes were pulled from borosilicate glass capillary tubing (1.5 mm outer diameter, 1.17 mm inner diameter; Clark Medical Instruments) to a tip of diameter approximately 1-1.5 μm and resistance of 2-4 MΩ. For single channel recordings pipettes were pulled from borosilicate glass capillary tubing (1.5 mm outer diameter, 0.8 mm inner diameter; Clark Medical Instruments) and fire polished before use. Voltage clamp commands were delivered via an Axopatch 1 D or Axon 200B patch clamp amplifiers (Axon Instruments) and membrane currents were recorded by a 12 bit AD/DA converter (Axodata 1200 or Labmaster, Scientific Solutions) interfaced to an Intel computer running pCLAMP software.

*Single Channel Bath Solutions: For free* $Ca^{2+}$ *less than 300nM:* All values in (mM): KCI, 140. Glucose, 10, EGTA 1 and HEPES, 10. *For free* $Ca^{2+}$ *greater than 300nM:* All values in (mM): KCI, 140. Glucose, 10, H-EDTA 1 and HEPES, 10. The same solution composition is used in the bath as in the patch pipette for these experiments except that the pipette solution contained 100 nM $Ca^{2+}$.

In single channel experiments voltage commands were applied using pClamp ramped potentials. This allowed more efficient measurement of slope conductance and channel activation than conventional step depolarisations. Activation curves were calculated by averaging current responses to 15 potential ramps and dividing each data point of the averaged current by the single channel amplitude at that holding potential, after leakage current correction. The rate of change of the applied ramp potentials were sufficiently slow (100mV$s^{-1}$) so that the activation curves were not distorted by the time constants of activation or deactivation. This analysis provides a continuous recording of the number of open channels multiplied by the open probability (NPo) over the entire voltage range. To obtain values for the steepness of the voltage-dependent activation and half-maximal activation voltage, activation curves were fitted with Boltzman functions of the form:

$$NPo = n/\{1+\exp[-K(V-V_{1/2})]\}$$

where N is the number of channels in the patch, n is the maximal NPo level, $K^{-1}$ is the steepness of the voltage-dependent activation (change in potential necessary to cause an e-fold increase in activation) and $V_{1/2}$ is the voltage at which there is half-maximal activation. Again, all of the experiments were carried out at 36 ± 1°C.

**Example 1: Single channel recordings using inside-out patch clamp technique from isolated smooth muscle cells of the rabbit bladder.**

When voltage ramps were applied to inside out patches and the cytosolic face of the patch was bathed in solutions containing 100 nM Ca2+, brief single channel openings were only observed at potentials positive to +50 mV. Referring to Figure 1A, 3 distinct single channel openings which each carried a maximum current of ~30 pA at +100 mV were apparent in this single sweep. These large single channel currents are a hallmark of large conductance $K^+$ (BK) channels. When the Ca2+ concentration at the cytosolic face of the patch was increased to 1uM (Figure 1 B), the number of channels opening increased and the channels activated at more negative potentials. Increasing the Ca2+ concentration further to 10 uM as shown in Figure 1C further enhanced the single channel activity so that up to 4 distinct single channel openings could be observed at potentials negative to - 100 mV. Note that the current is inward at voltages negative to 0 mV (reversal potential for $K^+$) and is outward at potentials postive to this. Panel D of Figure 1 shows summary data from these three experiments, and demonstrates that the voltage at which half of the channels in the patch were maximally activated V, (mV) shifted negatively with increasing concentrations of $Ca^{2+}$. In general, a 10 fold change in $Ca^{2+}$ shifted the V, by -100 mV consistent with the idea that these channels comprise the α and β subunits of the BK channel. Having established that these channels had a large conductance, were both voltage and calcium sensitive and the currents reversed at the K+ equilibrium potential (0 mV) we next tested the effect of the selective BK channel blocker Penitrem A in a separate series of experiments. Figure 1E shows a typcial example of an experiment in which single channel recordings were obtained in the presence of 1μM $Ca^{2+}$ using a voltage ramp protocol (upper panel) before (lower panel) application of the blocker. In this example, the BK channels activated at negative potentials (--50 mV) reversed at 0 mV and were macimally activated at positive potentials. Figure 1F shows the recording from same patch of membrane after application of the selective BK channel blocker Penitrem A (100 nM). As the figure suggests, Penitrem A abolished the single channel openings.

These data characterize the current under investigation, and are consistent with the characteristics that would be expected from a large conductance BK channel, showing that the current being activated is the current elicited by opening of BK channels.

**Example 2: Isolated cells using perforated patch with sodium 1-amino-9,10-dioxo-4-(phenylamino)-9,10-dihydroanthracene-2-sulfonate. (Acid Blue 25)**

Isolated smooth muscle cells are dispersed as described in Example 1. Currents were recorded using the perforated patch configuration of the whole cell patch clamp technique (Rae *et al.,* 1991). The cell membrane was perforated using the antibiotic amphotericin B (600 $\mu g.ml^{-1}$). Other methods including the preparation of patch pipettes and recording of currents are as described in Example 1.

*K Perforated Patch Solution:* All values in (mM): KCl, 132.96. $MgC1_2.6H_20$, 1. EGTA, 0.5 and HEPES, 10. Standard Hank's solution is used to bathe the cells in these experiments. Isolated smooth muscle cells from rabbit urethra we held at -60mV. The test protocol involved stepping from - 60mV to -80mV for 500msecs, and then stepping the voltage up in +10mV increments to +50mV before returning to the holding potential of -60mV. This is diagrammatically shown in Figure 2.

The Control panel in Figure 2 shows a family of outward currents recorded using the perforated patch configuration and elicited under in response to the voltage protocol shown in the lower panel. These noisy outward currents are consistent with currents being carried through large conductance BK channels. The centre panel of Figure 2 shows the potentiating effects of Acid Blue 25 (10μM) on the outward BK current in response to the same voltage steps. The right hand panel shows the effect of Acid Blue 25 can be washed out returning the currents to control levels. Acid Blue 25 (10μM) enhances the amplitude of the BK current compared to control, an effect that is reversible upon wash-out.

**Example 3: sodium 1-amino-9,10-dioxo-4-(3-(trifluoromethyl)phenylamino)-9,10-dihydroanthracene-2-sulfonate (SR-5-6) potentiates STOCs (Spontaneous Transient Outward Currents) recorded using the perforated patch clamp technique from isolated smooth muscle cells from the rabbit bladder.**

Using the perforated patch clamp techniques described in Example 2, the effects of the compounds of the present invention were observed on spontaneous transient outward currents (STOCs). In this experiment, isolated smooth muscle cells from rabbit bladder were held under voltage clamp at - 30mV and fired spontaneously active transient outward currents, as a result of activation of BK currents.

As seen in Figure 3, application of 1 0pM SR-5-6 increased the amplitude of STOCs, recorded using perforated patch clamp techniques. This effect was reversible on wash-out. These data indicate that SR-5-6 activates BK channels in spontaneously active smooth muscle cells when the cells are held at -30mV.

**Example 4: Dose-response of sodium 1-amino-9,10-dioxo-4-(3-(trifluoromethyl)phenylamino)-9,10-dihydroanthracene-2-sulfonate (SR-5-6) at single voltage step - Perforated patch clamp recordings from isolated smooth muscle cells from the rabbit bladder.**

Using the perforated patch clamp techniques described in Example 2, isolated smooth muscle cells from the rabbit bladder were held at -60mV and stepped to 0mV for 500 msec to elicit the BK current. As seen in Figure 4, the outward current elicited during perforated patch recordings using this protocol is potentiated in a dose dependent manner, using the compound sodium 1-amino-9,10-dioxo-4-(3-(trifluoromethyl)phenylamino)-9,10-dihydroanthracene-2-sulfonate (SR-5-6). These data demonstrate that the activation of BK channels by SR-5-6 occurs in a dose-dependent manner when the cell is exposed to the above protocol.

**Example 5: Effects of sodium 1-amino-9,10-dioxo-4(3-(trifluoromethyl)phenylamino)-9,10-dihydroanthracene-2-sulfonate (SR-5-6) on L-type Calcium channel - Perforated patch clamp recordings from isolated smooth muscle cells of the rabbit bladder, using $Cs^+$ in the pipette solution to block BK channels.**

Using methods described above herein, the patch pipette solution was altered to allow for measurement of inward L-type calcium currents which although present in earlier studies are not visible due to the overwhelming size of the outward BK current. By replacing $K^+$ with $Cs^+$ ions, the outward current is now blocked and a prominent inward current carried by Calcium is present. This is the L-type calcium ion channel currents allowing calcium into the cells.

*Pipette Solution:* All values in (mM): CsCI, 132.96. $MgCl_2.6H_20$, 1. EGTA, 0.5 and HEPES, 10.

With reference to Figure 5, isolated smooth muscle cells from the rabbit bladder were held at -60mV and stepped to 0mV for 500 ms. Pipette solutions contained $Cs^+$ to block outward $K^+$ currents. An inward L-type calcium current measured using perforated patch clamp technique, is reduced in amplitude in the presence of SR-5-6 in a dose-dependent manner. As the BK channels are known to be activated both by a change in voltage but also by an increase in intracellular $Ca^{2+}$ ions, it is important to rule out a mechanism of action whereby SR-5-6 was activating BK channels by increasing the influx of $Ca^{2+}$ through activation of voltage gated $Ca^{2+}$ channels and subsequent $Ca^{2+}$ activation of BK channels. This experiment shows that the increase in outward BK current with SR-5-6 observed previously (Figures 3-4) is not due to activation of L-type calcium currents and subsequent influx of calcium, rather that there is a modest inhibitory effect on influx of $Ca^{2+}$ through these channels.

**Example 6: Ramp protocol showing sodium 1-amino-9,10-dioxo-4-(3-(trifluoromethyl)phenylamino)-9,10-dihydroanthracene-2-sulfonate (SR-5-6) effects - Single channel recordings using inside-out patch clamp technique from isolated smooth muscle cells of the rabbit bladder.**

Using the single channel inside-out patch clamp technique described in Example 1, recordings of BK single channels in patches of membrane from isolated smooth muscle cells from rabbit bladder, were obtained using the inside out patch clamp configuration. The patches were held at a potential of - 100mV and voltage ramps were then applied from -100mV up to +100mV over 2s.

As seen in Figure 6, when $Ca^{2+}$ was buffered to -200 nM, the BK channels began to activate at potentials positive to +50mV. When the same voltage ramp was reapplied in the presence of 10μM SR5-6 the threshold voltage for activation of the channels was shifted -100 mV in the hyperpolarising direction.

The results of this experiment show that the current evoked in response to the voltage ramp protocol shifted the activation potential to a much more negative potential. This is consistent with the idea that this compound activates BK channels since having a greater population of BK channels open would cause activation of an outward BK current at more negative potentials in the physiologically relevant range of potentials (ie negative to 0 mV). In addition, the amount of current elicited throughout the duration of the ramp is larger in the presence of SR-5-6 than in control conditions.

Finally, since these data were collected from an inside-out patch, a situation where the compound is applied to the inside of the cell membrane, it seems probable that the compound is activating the BK channel on this small patch of membrane from the inside surface of the cell. Consequently one can conclude that SR-5-6 activates BK channels in an isolated patch of membrane, by directly opening the ion channel from the inside surface of the cell. However, given the data from whole cell recordings we can also conclude that the compound can activate BK channels when presented on exterior surface (outside) of the cell.

**Example 7: Cumulative data showing sodium 1-amino-9,10-dioxo-4-(3-(trifluoromethyl)phenylamino)-9,10-dihydroanthracene-2-sulfonate (SR-5-6) on ramps - Single channel recordings using inside-out patch clamp technique from isolated smooth muscle cells of the rabbit bladder.**

Using the single channel inside out patch clamp technique, described in Example 1, voltage ramps were used to observe openings of BK channels, as described in Example 6.

Referring to Figure 7, Panel A shows the control trace using the ramp protocol as described above herein. Panel B shows the effect of 10IJM SR-5-6 on the channel activity, as seen previously. Panel C shows a typical example of the effects of SR-5-6 on an inside out patch. To obtain these data, the average single channel currents were obtained from 15 voltage ramp sweeps under control conditions and then in the presence of each concentration of the drug. The mean currents were corrected for driving force by dividing the current by the single channel amplitude at each potential. Consequently, activation curves similar to those shown in Figure 7C can be obtained. When these data are fitted with a Boltzmann relationship, the voltage at which half of the channels in the patch are activated can be calculated ($V_{1/2}$). These data show that the effects of SR-5-6 are dose-dependent and shift the activation of the channels towards more physiologically relevant membrane potentials. Panel D shows a summary plot of six experiments in which the mean activation $V_{1/2}$ of the channels was plotted under control conditions (-200 nM $Ca^{2+}$) and in the presence of increasing concentrations of SR-5-6. The error bars show the standard error of the mean for each data point. These data show conclusively that the effects of SR-5-6 on the open probability and $\Delta V_{1/2}$ for BK channels are dose-dependent, which is a critical factor in the development of drug candidates.

**Example 8: Comparing structure function relationships using single channel recordings of inside-out patches from isolated smooth muscle cells of the rabbit bladder.**

To compare the effect of a variety of chemical substitutions on the BK channels, experiments were carried out using voltage ramps applied to inside out patches which were bathed with either 188 nM free Ca2+ or 100 nM free $Ca^{2+}$ on their cytosolic face. Patches were held at -100 mV and ramped through to +100 mV and each sweep was repeated 15 times. The same protocol was repeated after the patch was incubated in 1 $\mu$M $Ca^{2+}$. The patch was then returned to 100 nM $Ca^{2+}$ containing solutions and 10 $\mu$M of the drug of interest was applied. After a maximal effect was observed, the voltage ramps were reapplied to the patch in the presence of the drug. Data from each series of voltage ramp was then averaged experiment was then averaged. These mean currents were corrected for driving force by dividing the current by the single channel amplitude at each potential. When these data were fitted with a Boltzmann relationship, the voltage at which half of the channels in the patch were activated could be calculated ($V_{1/2}$). The observed shift in activation ($\Delta V_{1/2}$) under control conditions and in the presence of each drug was obtained by subtracting the $V_{1/2}$ in control and the $V_{1/2}$ in drug. Table 1 shows a summary data series of experiments in which the average $\Delta V_{1/2}$ of each molecule is compared.

**Table 1: The effect $\Delta V_{1/2}$ of activation in response to application of 10 $\mu$M of compounds according to a first aspect of the present invention**

| Compound | $\Delta V_{1/2}$ (mV) in 100 nm $Ca^{2+}$ |
|---:|---:|
| **SR-5-18** | -23.2 |
| **SR-5-14** | -24.2 |
| **SR-5-8** | -24.24 |
| **SR-5-15** | -28.4 |
| **SR-5-29** | -39.6 |
| **Acid Blue 25** | -51.0 |
| **SR-5-26** | -53.5 |

(continued)

| Compound | $\Delta V_{1/2}$ (mV) in 100 nm $Ca^{2+}$ |
|---|---|
| **SR-5-12** | -54.1 |
| **Acid Blue 62** | -54.37 |
| **SR-5-25** | -55.3 |
| **SR-5-32** | -61.1 |
| **SR-5-37** | -77.8 |
| **SR-5-28** | -83.2 |
| **SR-5-34** | -84.0 |
| **SR-5-31** | -87.1 |
| **SR-5-6** | -90.9 |
| **SR-5-40** | -97.8 |
| **SR-5-44** | -145.4 |

**Materials & Methods**

**General Details**

$^1$H-NMR and $^{13}$C-NMR data were collected at 300 K using a Bruker AMX 400 MHz NMR spectrometer at 400 MHz ($^1$H), or 100 MHz ($^{13}$C) , respectively. Residual DMSO (δ 2.50) was used as internal references for $^1$H NMR spectra. The data reported as chemical shift ($\delta_H$ ppm), relative integral, multiplicity (s = singlet, br = broad, d = doublet, t = triplet, q = quartet, m = multiplet), coupling constant (J Hz), and assignment. Solvent peak for DMSO (δ 39.7) was used as internal reference for $^{13}$C NMR spectra. High Resolution Mass Spectra (HRMS) was recorded by the School of Chemistry and Chemical Biology, University College Dublin using a Micromass/Waters LCT instrument. Microwave reactions were carried out using a CEM focused ™ Microwave Synthesis type Discover® apparatus. Reactions were monitored by thin layer chromatography (TLC), which was performed on aluminum sheets pre-coated with Silica gel 60 $F_{254}$ (Merck). Column-chromatography separations were performed using Merck Kieselgel 60 (0.040-0.063 mm). The columns were usually eluted with various combinations of ethyl acetate-methanol mixtures. All reagents were obtained from commercial sources and used as received.

**Synthetic Procedure of Ullmann Coupling reaction**

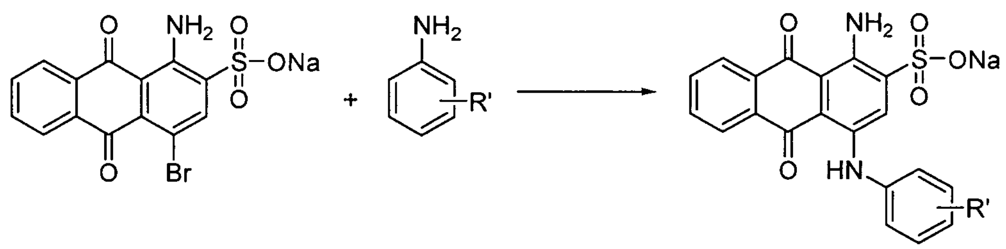

Procedure A: The suspension of copper powder (31 mg, 0.495 mmol) and buffer solution of $Na_2HP0_4$ (0.2 M, 3 mL) and $NaH_2PO_4$ (0.12 M, 5 mL) was sonicated for 20 minutes in a 35 mL microwave reaction vial. Bromaminic acid sodium salt (0.2 g, 0.495 mmol) and the suitable aniline derivative (0.99 mmol) were then added into that. The mixture was capped and irradiated in the microwave oven (70 W-80 W) with stirring for 5-20 minutes at 100- 120 °C. Reaction mixture was cooled to room temperature and filtered. Filtrate was extracted with ethyl acetate (40 mL x 3) and washed with water. Combined organic layer was dried over anhydrous sodium sulfate and concentrated under reduced pressure. The resulting crude product was purified by column chromatography on silica gel (4% methanol in ethyl acetate) to furnish the corresponding anilinoanthraquinone derivative as a blue powder.

**Table 2: Isolated yields of material judged homogeneous by TLC and NMR.**

| Compound | Yield (%)[a] |
|---|---|
| Acid Blue 25 | - |
| SR-5-6 | 75 |
| SR-5-11 | 43 |
| SR-5-12 | 83 |
| SR-5-13 | 30 |
| SR-5-14 | 36 |
| SR-5-15 | 51 |
| SR-5-18 | 25 |
| SR-5-23 | 41 |
| SR-5-25 | 32 |
| SR-5-26 | 82 |
| SR-5-27 | 33 |
| SR-5-28 | 65 |
| SR-5-29 | 36 |
| SR-5-31 | 79 |
| SR-5-34 | 76 |
| SR-5-37 | 68 |
| SR-5-40 | 79 |
| SR-5-44 | 75 |
| SR-5-46 | 62 |
| SR-5-47 | 44 |

[a]Yields reported here are isolated yields of material judged homogeneous by TLC and NMR.

Procedure B: Bromaminic acid sodium salt (0.2 g, 0.495 mmol), sodium carbonate (42 mg, 0.396 mmol), copper sulfate (16 mg, 0.1 mmol) and the suitable aniline or amine derivative were mixed in 5 mL water. Reaction mixture was stirred at 65 °C for 1 h and then refluxed gently at 105 °C for 5 h. The reaction mixture was then cooled to room temperature and filtered. Filtrate was extracted with ethyl acetate (40 mL × 3) and washed with water. Combined organic extract was dried over anhydrous sodium sulfate and concentrated under reduced pressure. The resulting crude product was loaded on silica gel column and eluted with 3% methanol in ethyl acetate to obtain the corresponding anilinoanthraquinone derivative as a blue powder.

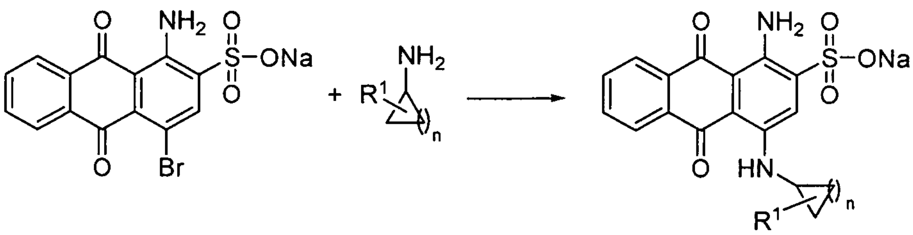

**Table 3: isolated yields of material judged homogeneous by TLC and NMR**

| n | Compound | Yield (%)[a] |
|---|---|---|
| 1 | SR-5-10 | 39 |
| 3 | SR-5-8 | 47 |
| 4 | Acid blue 62 | - |

(continued)

| n | Compound | Yield (%)[a] |
|---|---|---|
| 5 | SR-5-32 | 49[b] |

[a]Yields reported here are isolated yields of material judged homogeneous by TLC and NMR.
[b]Reaction was performed following the procedure B.

SR-5-6: sodium 1-amino-9,10-dioxo-4-(3-(trifluoromethyl)phenylamino)-9,10-dihydroanthracene-2-sulfonate.

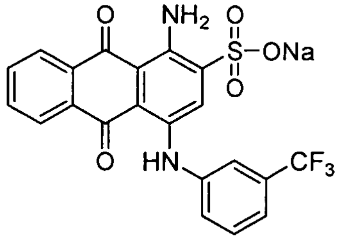

$R_f$: 0.4 (3 % methanol/ ethyl acetate).
$^1$H NMR (400 MHz, DMSO-$d_6$): δ 11.80 (s, 1H), 10.02 (brs, 1H), 8.24 (dd, J = 7.2, 13.2 Hz, 2H), 8.04 (s, 1 H), 7.85 (m, 2H), 7.65-7.56 (m, 3H), 7.48 (d, J = 7.6 Hz, 1H).
$^{13}$C NMR (100 MHz, DMSO-$d_6$): δ 183.2, 182.1, 144.6, 142.2 (2C), 140.8, 138.8, 134.0, 133.4, 133.3, 132.9, 130.7 (2C), 126.0, 125.9, 125.6, 122.8, 119.9, 118.4, 113.1, 109.6.
HRMS (ES): m/z for $C_{21}H_{12}N_2O_5F_3S$ [M - $Na^+$], calcd. 461.0419, found 461.0440.

SR-5-8: sodium 1-amino-4-(cyclopentylamino)-9,10-dioxo-9,10-dihydroanthracene-2-sulfonate.

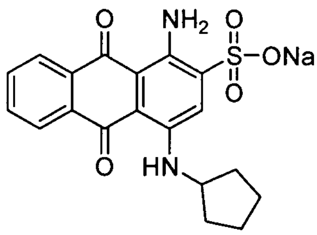

$R_f$: 0.4 (3 % methanol/ ethyl acetate).
$^1$H NMR (400 MHz, DMSO-$d_6$): 5 10.86 (d, J= = 6.8 Hz, 1H), 10.13 (brs, 1H), 8.27-8.23 (m, 2H), 7.82 (s, 1H), 7.81-7.79 (m, 2H), 4.14-4.10 (m, 1H), 2.14-2.07 (m, 2H), 1.80-1.68 (m, 6H).
$^{13}$C NMR (100 MHz, DMSO-$d_6$): δ 181.5, 180.7, 144.7, 143.4, 143.0, 133.9 (2C), 132.5, 132.4, 125.8, 125.7, 121.6, 109.0, 108.7, 53.3, 33.6 (2C), 23.6 (2C).
HRMS (ES): m/z for $C_{19}H_{18}N_2O_sNaS$ [M + $H^+$], calcd. 409.0834, found 409.0850.

SR-5-10: sodium 1-amino-4-(cyclopropylamino)-9,10-dioxo-9,10-dihydroanthracene-2-sulfonate.

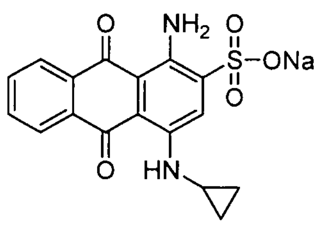

$R_f$: 0.4 (4 % methanol/ ethyl acetate).

$^1H$ NMR (400 MHz, DMSO-$d_6$): δ 10.51 (d, J = 2.4 Hz, 1 H), 10.09 (brs, 1 H), 8.27-8.24 (m, 1 H), 8.22-8.20 (m, 1H), 8.17 (s, 1H), 7.82-7.80 (m, 2H), 2.80-2.67 (m, 1H), 0.94-0.90 (m, 2H), 0.65-0.62 (m, 2H).
$^{13}C$ NMR (100 MHz, DMSO-*$d_6$*): δ 181.7, 181.4, 145.8, 143.4, 143.2, 133.9, 133.7, 132.6 (2C), 125.9, 125.7, 122.1, 109.2, 109.1, 24.1, 7.63 (2C).
HRMS (ES): m/z for $C_{17}H_{l3}N_2O_5Na_2S$ [M + $Na^+$], calcd. 403.0341, found 403.0359.

SR-5-11: sodium 1-amino-4-(2,6-difluorophenylamino)-9,10-dioxo-9,10-dihydroanthracene-2-sulfonate.

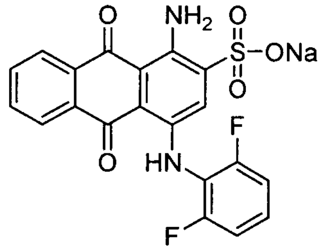

$R_f$: 0.4 (4 % methanol/ ethyl acetate).
$^1H$ NMR (400 MHz, DMSO-$d_6$): δ 11.52 (s, 1H), 9.98 (brs, 1H), 8.30-8.27 (m, 2H), 7.89-7.86 (m, 2H), 7.43-7.30 (m, 4H).
$^{13}$ C NMR (100 MHz, DMSO-$d_6$): δ 183.5, 182.1, 158.91-156.45 (d, J = 246.0 Hz), 158.87-156.40 (d, J = 247.0 Hz), 144.1, 142.7, 140.6, 134.1, 133.5, 133.3, 132.9, 127.4, 126.1, 126.0, 121.8, 115.91-115.75 (d, J = 16.0 Hz), 112.6, 112.4, 111.5, 109.2.
HRMS (ES): m/z for $C_{2o}H_{11}N_2O_sF_2S$ [M - $Na^+$], calcd. 429.0357, found 429.0348.

SR-5-12: sodium 1-amino-4-(2-methoxyphenylamino)-9,10-dioxo-9,10-dihydroanthracene-2-sulfonate.

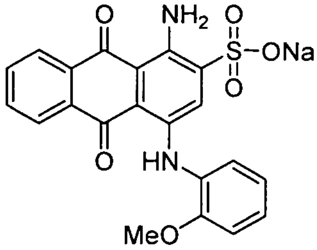

$R_f$: 0.3 (4 % methanol/ ethyl acetate).
$^1H$ NMR (400 MHz, DMSO-$d_6$): δ 11.94 (s, 1 H), 10.12 (brs, 1H), 8.30-8.27 (m, 2H), 7.97 (s, 1 H), 7.88-7.83 (m, 2H), 7.30 (d, J = 7.2 Hz, 1H), 7.20-7.17 (m, 2H), 7.05-7.01 (m, 1H), 3.88 (s, 3H).

SR-5-13: sodium 4-(3,5-bis(trifluoromethyl)phenylamino)-1-amino-9,10-dioxo-9,10-dihydroanthracene-2-sulfonate.

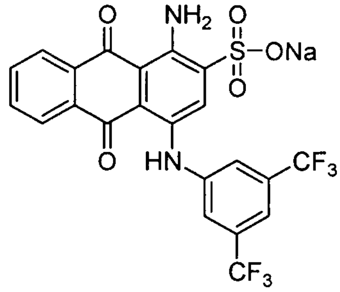

$R_f$: 0.4 (4 % methanol/ ethyl acetate).
$^1H$ NMR (400 MHz, DMSO-*$d_6$*): δ 11.39 (s, 1H), 9.87 (brs, 1H), 8.21-8.14 (m, 2H), 7.98 (s, 1H), 7.85-7.78 (m, 4H), 7.62

(s, 1H).
HRMS (ES): m/z for $C_{22}H_{11}N_2O_5F_6S$ [M - $Na^+$], calcd. 529.0293, found 529.0292.

SR-5-14: sodium 1-amino-4-(3,5-difluorophenylamino)-9,10-dioxo-9,10-dihydroanthracene-2-sulfonate.

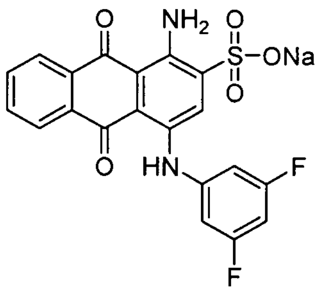

$R_f$: 0.4 (4 % methanol/ ethyl acetate).
$^1$H NMR (400 MHz, DMSO-$d_6$): δ 11.55 (s, 1H). 9.98 (brs, 1H), 8.28-8.22 (m, 2H), 8.08 (s, 1H), 7.91-7.84 (m, 2H), 7.49 (brs, 1 H), 7.00 (dd, J = 2.4, 9.4 Hz, 2H), 6.96-6.90 (m, 1 H).
HRMS (ES): m/z for $C_{2o}H_{11}N_2O_sF_2S$ [M - $Na^+$], calcd. 429.0357, found 429.0370.

SR-5-15: sodium 1-amino-4-(3-fluorophenylamino)-9,10-dioxo-9,10-dihydroanthracene-2-sulfonate.

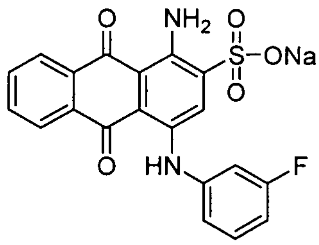

$R_f$: 0.4 (4 % methanol/ ethyl acetate).
$^1$H NMR (400 MHz, DMSO-$d_6$): δ 11.83 (s, 1 H), 10.05 (brs, 1 H), 8.28-8.23 (m, 2H), 8.06 (s, 1 H), 7.89-7.82 (m, 2H), 7.49-7.43 (m,1H). 7.17-7.11 (m, 2H), 6.99 (dt, *J*= 2.4,8.2 Hz, 1H).
$^{13}$ C NMR (100 MHz, DMSO-$d_6$): δ 183.0, 182.0, 164.05-161.63 (d, J = 242.0 Hz), 144.5, 142.3, 141.6, 139.2, 134.1, 133.4, 133.3, 132.9, 131.2, 126.1, 126.0, 123.0, 118.1, 112.6, 110.52-110.31 (d, J = 21.0 Hz), 109.4, 109.0.
HRMS (ES): m/z for $C_{20}H_{12}N_2O_5FS$ [M - $Na^+$], calcd. 411.0451, found 411.0464.

SR-5-18: sodium 1-amino-9,10-dioxo-4-(3-sulfophenylamino)-9,10-dihydroanthracene-2-sulfonate.

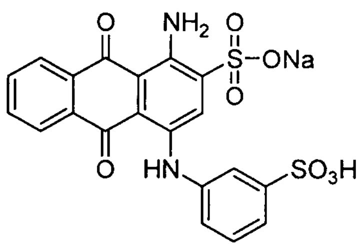

$R_f$: 0.3 (12 % methanol/ ethyl acetate).
$^1$H NMR (400 MHz, DMSO-$d_6$): δ 12.05 (s, 1H), 10.11 (brs, 1 H), 8.30-8.28 (m, 2H), 8.00 (s, 1H), 7.88-7.85 (m, 2H), 7.46-7.39 (m, 3H), 7.25 (td, J = 2.4, 7.1 Hz, 1H).

SR-5-23: sodium 1-amino-4-(3-fluoro-5-(trifluoromethyl)phenylamino)-9,10-dioxo-9,10-dihydroanthracene-2-sulfonate.

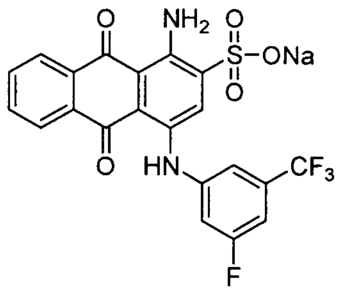

$R_f$: 0.4 (4 % methanol/ ethyl acetate).
$^1H$ NMR (400 MHz, DMSO-$d_6$): δ 11.51 (s, 1H), 9.96 (brs, 1 H), 8.25 (dd, J = 3.2, 15.6 Hz, 2H), 8.06 (s, 1H), 7.95-7.84 (m, 2H), 7.47 (s, 1H), 7.42 (d, J = 10.8 Hz, 1H), 7.32 (d, J = 8.0 Hz, 1H).
HRMS (ES): m/z for $C_{21}H_{11}N_2O_5F_4S$ [M - $Na^+$], calcd. 479.0325, found 479.0344.

SR-5-25: sodium 1-amino-9,10-dioxo-4-(2-(trifluoromethyl)phenylamino)-9,10-dihydroanthracene-2-sulfonate.

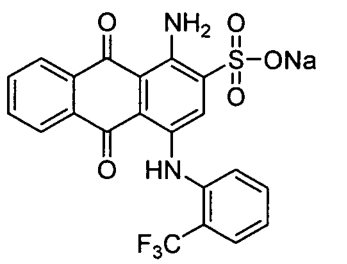

$R_f$: 0.4 (3 % methanol/ ethyl acetate).

SR-5-26: sodium 4-(m-toluidino)-1-amino-9,10-dioxo-9,10-dihydroanthracene-2-sulfonate.

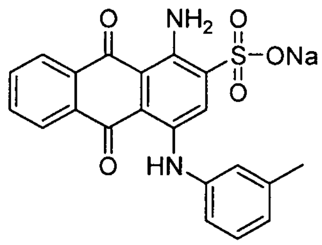

$R_f$: 0.4 (3 % methanol/ ethyl acetate).
$^1H$ NMR (400 MHz, DMSO-$d_6$): δ 12.05 (s, 1H), 10.12 (brs, 1H), 8.27 (dt, J = 2.4, 6.8 Hz, 2H), 8.04 (s, 1H), 7.87-7.82 (m, 2H), 7.54 (brs, 1 H), 7.34 (t, J = 7.6 Hz, 1 H), 7.11 (s, 1H), 7.07 (dd, J = 7.6, 24.4 Hz, 2H), 2.35 (s, 3H).

SR-5-27: sodium 1-amino-4-(3-fluoro-4-(trifluoromethyl)phenylamino)-9,10-dioxo-9,10-dihydroanthracene-2-sulfonate.

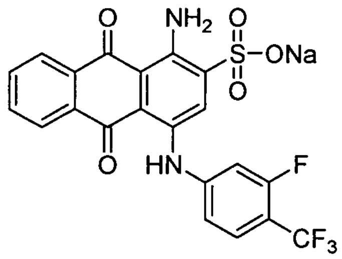

$R_f$: 0.4 (3 % methanol/ ethyl acetate).
$^1$H NMR (400 MHz, DMSO-$d_6$): δ 11.36 (s, 1 H), 9.93 (brs, 1 H), 8.27-8.21 (m, 2H), 8.11 (s, 1 H), 7.92-7.84 (m, 2H), 7.72 (t, J = 4.8 Hz, 1H),7.31 (d, J = 13.6 Hz, 1 H), 7.18 (d, J = 8.6 Hz, 1 H).
HRMS (ES): m/z for $C_{21}H_{11}N_2O_sF_4Na_2S$ [M + $Na^+$], calcd. 525.0120, found 525.0125.

SR-5-28: sodium 1-amino-4-(4-fluoro-3-(trifluoromethyl)phenylamino)-9,10-dioxo-9,10-dihydroanthracene-2-sulfonate.

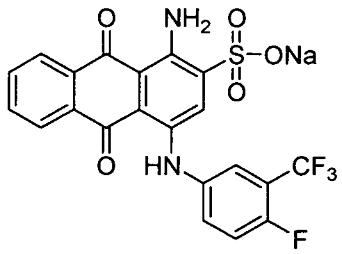

$R_f$: 0.4 (3 % methanol/ ethyl acetate).
$^1$H NMR (400 MHz, DMSO-$d_6$): δ 11.73 (s, 1H), 9.99 (brs, 1H), 8.21 (dd, J = 7.2, 15.2 Hz, 2H), 7.91 (s, 1H), 7.86-7.80 (m, 2H), 7.67-7.63 (m, 2H), 7.57 (t, J = 10.0 Hz, 1H).
$^{13}$ C NMR (100 MHz, DMSO-$d_6$): 183.0, 182.0, 156.37-153.87 (d, J = 250.0 Hz), 144.4, 142.3, 139.5, 136.7, 136.6, 134.0, 133.3 (2C), 132.9, 129.0, 126.0, 125.9, 122.4, 121.3, 121.2, 118.50-118.28 (d, J = 22.0 Hz), 112.7, 109.5.
HRMS (ES): m/z for $C_{21}H_{11}N_2O_5F_4Na_2S$ [M + $Na^+$], calcd. 525.0120, found 525.0107.

SR-5-29: sodium 1-amino-9,10-dioxo-4-(4-(trifluoromethyl)phenylamino)-9,10-dihydroanthracene-2-sulfonate.

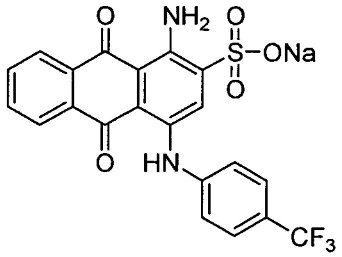

$R_f$: 0.4 (4 % methanol/ ethyl acetate).

SR-5-31: sodium 1-amino-4-(3-ethylphenylamino)-9,10-dioxo-9,10-dihydroanthracene-2-sulfonate.

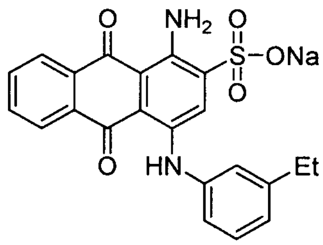

$R_f$: 0.4 (3 % methanol/ ethyl acetate).
**$^1$H** NMR (400 MHz, DMSO-$d_6$): δ 12.07 (s, 1H). 10.13 (brs, 1H), 8.31-8.23 (m, 2H), 8.08 (s, 1H), 7.87-7.81 (m, 2H), 7.36 (t, J = 7.6 Hz, 1H), 7.14 (s, 1H), 7.08 (dd, J = 8.0, 20.2 Hz, 2H), 2.64 (ABq, J = 7.6 Hz, 2H), 1.22 (t, J = 7.6 Hz, 3H) .
$^{13}$ C NMR (100 MHz, DMSO-$d_s$): 182.3, 181.8, 145.6, 144.3, 142.6, 140.9, 139.1, 134.1, 133.5, 133.1, 132.7, 129.5, 126.0, 125.9, 124.0, 122.8, 122.5, 120.3, 111.3, 109.1, 28.1, 15.5.
HRMS (ES): m/z for $C_{22}H_{l7}N_2O_5Na_2S$ [M + $Na^+$], calcd. 467.0654, found 467.0666.

SR-5-32: sodium 1-amino-4-(cycloheptylamino)-9,10-dioxo-9,10-dihydroanthracene-2-sulfonate.

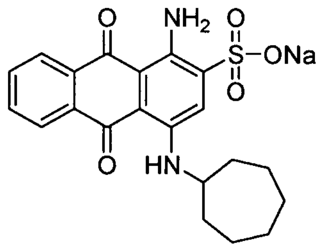

$R_f$: 0.4 (3 % methanol/ ethyl acetate).
$^1$H NMR (400 MHz, DMSO-$d_6$): δ 10.95 (d, J = 7.6 Hz, 1 H), 10.18 (brs, 1 H), 8.27-8.24 (m, 2H), 7.81-7.79 (m, 2H), 7.75 (s, 1H), 3.91-3.86 (m, 1H), 2.02-1.96 (m, 2H), 1.70-1.59 (m, 10H).
$^{13}$C NMR (100 MHz, DMSO-$d_6$): δ 181.4, 180.6, 144.2, 143.5, 143.0, 134.0, 133.9, 132.4, 132.3, 125.8, 125.7, 121.4, 109.1, 108.7, 52.1, 34.7 (2C), 27.6 (2C), 23.5 (2C).
HRMS (ES): m/z for $C_{21}H_{21}N_20_5Na_2S$ [M + $Na^+$], calcd. 459.0967, found 459.0970.

SR-5-34: sodium 1-amino-9,10-dioxo-4-(3-(trifluoromethoxy)phenylamino)-9,10-dihydroanthracene-2-sulfonate.

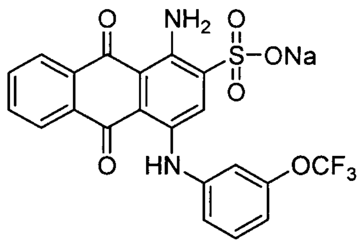

$R_f$: 0.4 (3 % methanol/ ethyl acetate).
$^1$H NMR (400 MHz, DMSO-$d_6$): δ 11.78 (s, 1H), 10.02 (brs, 1 H), 8.27-8.22 (m, 2H), 8.07 (s, 1 H), 7.89-7.82 (m, 2H), 7.54 (t, J = 8.0 Hz, 1H), 7.31-7.28 (m, 2H), 7.12 (d, J = 8.4 Hz, 1 H).
$^{13}$ C NMR (100 MHz, DMSO-$d_6$): δ 183.2, 182.1, 149.3, 144.6, 142.2, 141.7, 138.7, 134.0, 133.4, 133.3, 132.9, 131.2 (2C), 126.0 (2C), 123.0, 120.6, 115.6, 114.3, 113.1, 109.6.
HRMS (ES): m/z for $C_{21}H_{12}N_20_sF_3Na_2S$ [M + $Na^+$], calcd. 523.0164, found 523.0179.

SR-5-37: sodium 1-amino-4-(4-benzylphenylamino)-9,10-dioxo-9,10-dihydroanthracene-2-sulfonate.

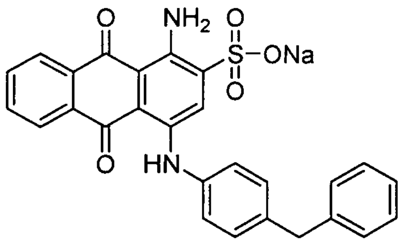

$R_f$: 0.4 (3 % methanol/ ethyl acetate).
' H NMR (400 MHz, DMSO-$d_s$): δ 12.07 (s, 1 H), 10.11 (brs, 1 H), 8.29-8.26 (m, 2H), 8.00 (s, 1 H), 7.88-7.81 (m, 2H), 7.34-7.20 (m, 9H), 3.98 (s, 2H).
HRMS (ES): m/z for $C_{27}H_{l9}N_20_5Na_2S$ [M + $Na^+$], calcd. 529.0810, found 529.0812.

SR-5-40: sodium 1 -amino-4-(4-chloro-3-(trifluoromethyl)phenylamino)-9,10-dioxo-9,10-dihydroanthracene-2-sulfonate.

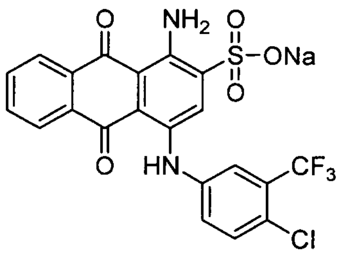

$R_f$: 0.4 (4 % methanol/ ethyl acetate).
$^1$H NMR (400 MHz, DMSO-$d_6$): δ 11.61 (s, 1 H), 9.96 (brs, 1H), 8.24-8.17 (m, 2H), 8.00 (s, 1H), 7.87-7.80 (m, 2H), 7.72 (s, 1 H), 7.71 (d, J = 12.0 Hz, 1 H), 7.54 (dd, J = 2.8, 8.8 Hz, 1 H).
HRMS (ES): m/z for $C_{21}H_{11}N_20_sF_3Na_2SCI$ [M + $Na^+$], calcd. 540.9825, found 540.9824.

SR-5-44: sodium 1-amino-4-(4-methyl-3-(trifluoromethyl)phenylamino)-9,10-dioxo-9,10-dihydroanthracene-2-sulfonate.

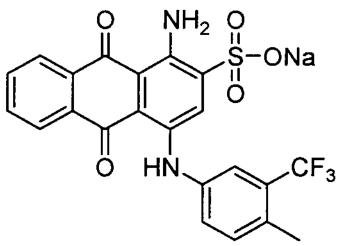

$R_f$: 0.4 (3 % methanol/ ethyl acetate).
$^1$H NMR (400 MHz, DMSO-$d_6$): δ 11.85 (s, 1 H), 10.05 (brs, 1 H), 8.25-8.20 (m, 2H), 7.97 (s, 1 H), 7.86-7.80 (m, 2H), 7.55 (s, 1H), 7.49-7.44 (m, 2H), 2.45 (s, 3H).
HRMS (ES): m/z for $C_{22}H_{14}N_20_sF_3Na_2S$ [M + $Na^+$], calcd. 521.0371, found 521.0382.

SR-5-46: sodium 1-amino-4-(3-chlorophenylamino)-9,10-dioxo-9,10-dihydroanthracene-2-sulfonate.

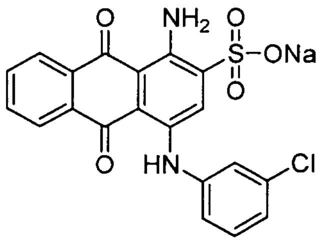

$R_f$: 0.4 (4 % methanol/ ethyl acetate).

$^1$H NMR (400 MHz, DMSO-$d_6$)$_1$ δ 11.79 (s, 1H), 10.04 (brs, 1 H), 8.28-8.23 (m, 2H), 8.03 (s, 1 H), 7.89-7.83 (m, 2H), 7.45 (t, J = 8.0 Hz, 1 H), 7.36 (t, J = 2.0 Hz, 1H), 7.26-7.20 (m, 2H).

SR-5-47: sodium 1-amino-4-(3-cyanophenylamino)-9,10-dioxo-9,10-dihydroanthracene-2-sulfonate.

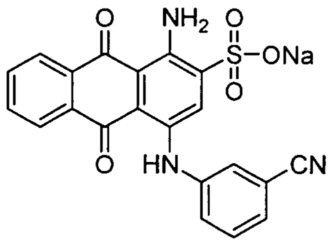

$R_f$: 0.3 (4 % methanol/ ethyl acetate).

$^1$H NMR (400 MHz, DMSO-$d_6$): δ 11.72 (s, 1 H), 10.01 (brs, 1 H), 8.28-8.23 (m, 2H), 7.99 (s, 1 H), 7.90-7.83 (m, 2H), 7.74 (s, 1H), 7.62-7.56 (m, 3H).

HRMS (ES): m/z for $C_{21}H_{12}N_30_sNa_2S$ [M + $Na^+$], calcd. 464.0293, found 464.0280.

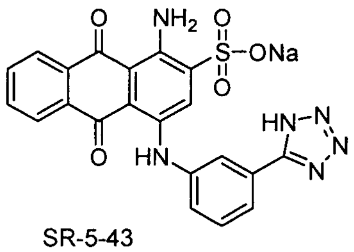

SR-5-43

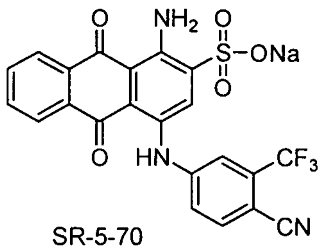

SR-5-70

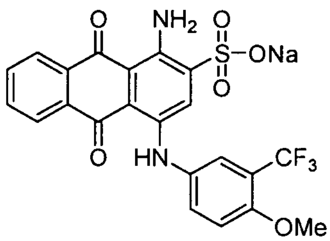

SR-5-71

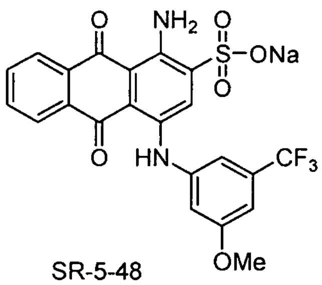

SR-5-48

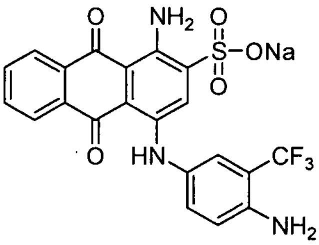

SR-5-45

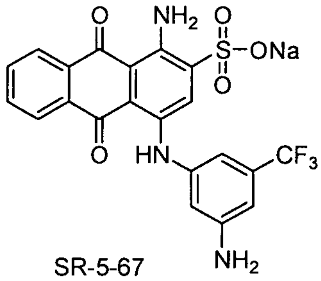

SR-5-67

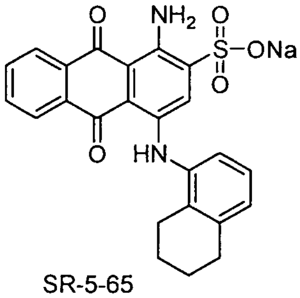

SR-5-65

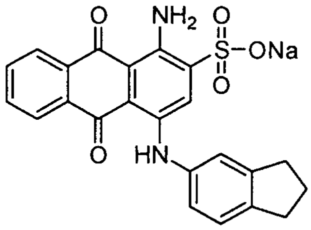

SR-5-64

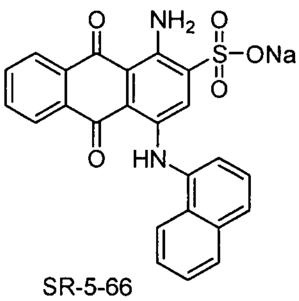

SR-5-66

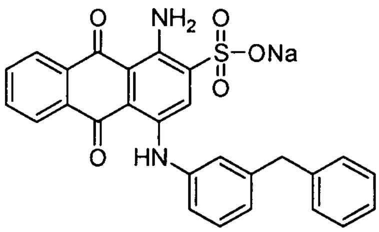

SR-5-68

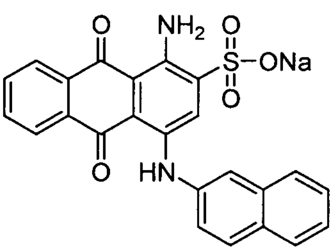

SR-5-72

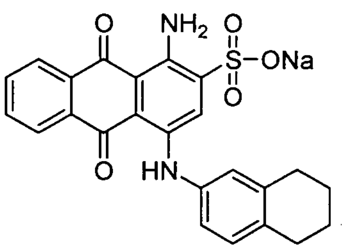

SR-5-69

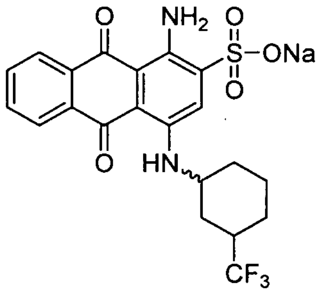

SR-5-73

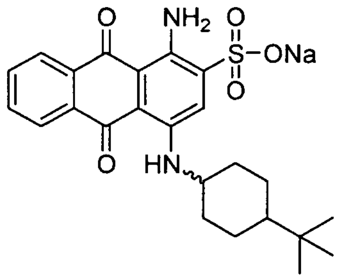

SR-5-74

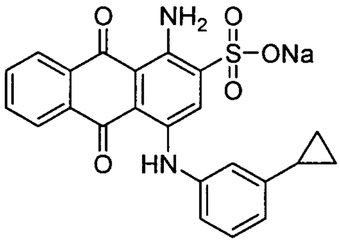

SR-5-75

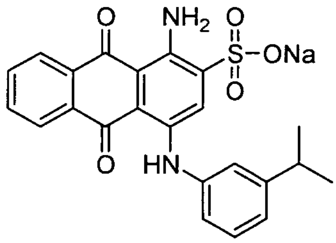

SR-5-63

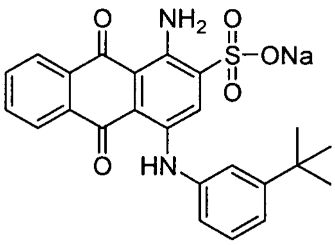

SR-5-76

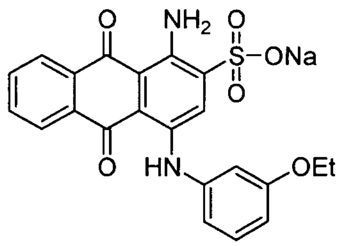

SR-5-77

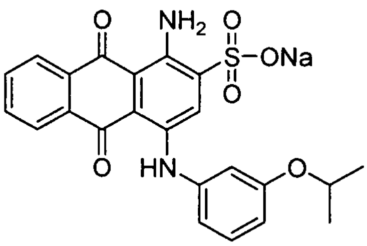

SR-5-78

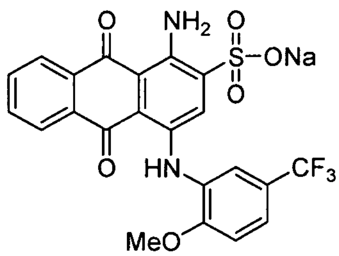

SR-5-61

## Claims

1. A compound comprising a substituted or unsubstituted anthraquinone, or a salt or isomer thereof, for use in treating

a disorder caused by or associated with dysfunctional ion channel activity; optionally wherein the ion channel is a potassium ion channel, further optionally a calcium-activated potassium ion channel, still further optionally a BK Channel.

**2.** A compound for use according to Claim 1, wherein the compound comprises an acid dye, or a salt or isomer thereof, which can be substituted or unsubstituted; optionally wherein the compound comprises an acid dye selected from an acid blue dye and an acid green dye, or a salt or isomer each thereof, which can be substituted or unsubstituted.

**3.** A compound for use according to Claim 1 or 2, wherein the compound has the general formula (I):

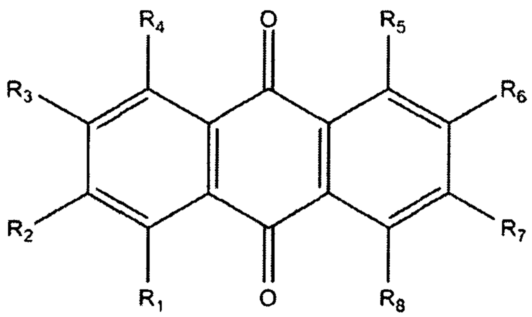

**(I)**

wherein;
$R_1$-$R_4$ and $R_7$ are each a hydrogen atom;
$R_5$ and $R_8$, which can be the same or different, are each independently selected from a hydrogen atom; and an amine, optionally a secondary amine; and
$R_6$ is independently selected from a hydrogen atom or an alkyl group; further optionally a sulfonate or a carboxyl group.

**4.** A compound for use according to any one of Claims 1-3, wherein the compound comprises a substituted anthraquinone and has the general formula (IA):

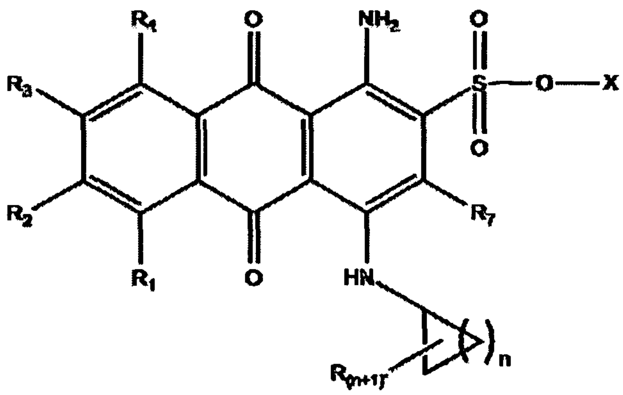

**(II)**

wherein;
n is an integer from 1 to 5;
$R_1$-$R_4$ and $R_7$ are each a hydrogen atom;
X is an alkali metal cation, optionally sodium;
each $R_{(n+1)'}$is independently selected from at least one of:

i. a hydrogen atom;
ii. a heteroatom selected from a halide, optionally fluoride or chloride; an oxygen atom; or an amine, optionally a primary amine;
iii. a short chain alkyl, alkenyl, or alkynyl group, which can be branched or unbranched, substituted or unsubstituted, linear or cyclic; optionally a short chain alkyl; further optionally an ethyl, propyl, or a butyl group;
iv. a short chain aza-alkyl, aza-alkenyl, or aza-alkynyl group, which can be branched or unbranched, substituted or unsubstituted, linear or cyclic;
v. a short chain alkoxyl, alkenoxyl, or alkynoxyl group, which can be branched or unbranched, substituted or unsubstituted, linear or cyclic;
vi. a short chain thio-alkyl, thio-alkenyl, or thio-alkynyl group, which can be branched or unbranched, substituted or unsubstituted, linear or cyclic;
vii. a short chain halo-alkyl, halo-alkenyl, or halo-alkynyl group, which can be branched or unbranched, substituted or unsubstituted, linear or cyclic; optionally a fluorinated methyl group; further optionally a trifluoromethyl group;
viii. a nitrile, hydroxyl or ester group;
ix. a sulfonate or carboxyl group; and
x. a tetrazole.

or a pharmaceutically acceptable salt, hydrate, or isomer thereof.

**5.** A compound for use according to Claim 4, wherein n is 5, and each $R_{(n-1)'}$ is a hydrogen atom; optionally wherein the compound is sodium 1-amino-4-(cycloheptylamino)-9,10-dioxo-9,10-dihydroanthracene-2-sulfonate.

**6.** A compound for use according to according to Claim 4, wherein n is 4, and optionally the compound has the general formula (IB):

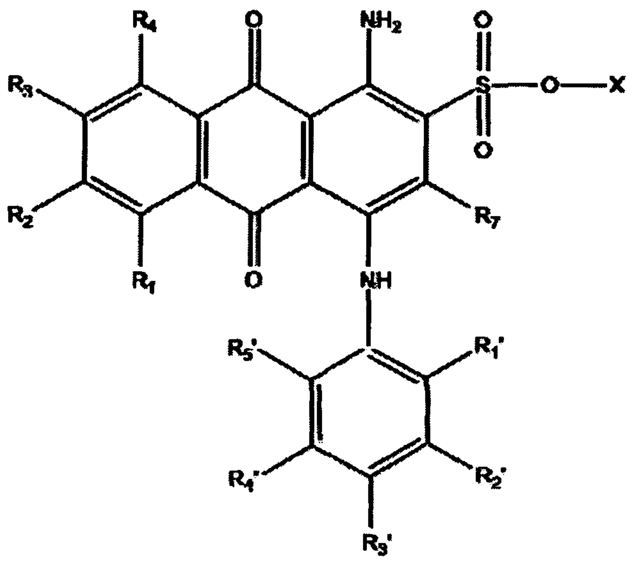

(IB)

wherein;
$R_{1'}$ and $R_5$', which can be the same or different, are each independently selected from at least one of each of:

i. a hydrogen atom;
ii. a heteroatom selected from a halide, optionally fluoride or chloride; or an oxygen atom;
iii. a short chain alkyl, alkenyl, or alkynyl group, which can be branched or unbranched, substituted or unsubstituted, linear or cyclic; optionally a short chain alkyl; further optionally an ethyl group;
iv. a short chain alkoxyl, alkenoxyl, or alkynoxyl group, which can be branched or unbranched, substituted or unsubstituted, linear or cyclic; and
v. a short chain halo-alkyl, halo-alkenyl, or halo-alkynyl group, which can be branched or unbranched, substituted or unsubstituted, linear or cyclic;

$R_{2'}$ and $R_{4'}$, which can be the same or different, are each independently selected from at least one of each of:

i. a hydrogen atom;
ii. a heteroatom selected from a halide, optionally fluoride or chloride; an oxygen atom; or an amine, optionally

a primary amine;
iii. a short chain halo-alkyl, halo-alkenyl, or halo-alkynyl group, which can be branched or unbranched, substituted or unsubstituted, linear or cyclic;
iv. a sulfonate or carboxyl group;
v. a short chain alkyl, alkenyl, or alkynyl group, which can be branched or unbranched, substituted or unsubstituted, linear or cyclic;
vi. a nitrile group;
vii. a short chain alkoxyl, alkenoxyl, or alkynoxyl group, which can be branched or unbranched, substituted or unsubstituted, linear or cyclic; and
viii. a tetrazole; and

$R_{3'}$ is independently selected from at least one of each of:

i. a hydrogen atom;
ii. a short chain halo-alkyl, halo-alkenyl, or halo-alkynyl group, which can be branched or unbranched, substituted or unsubstituted, linear or cyclic;
iii. a heteroatom selected from a halide, optionally fluoride or chloride; an oxygen atom; or an amine, optionally a primary amine;
iv. a short chain alkyl, alkenyl, or alkynyl group, which can be branched or unbranched, substituted or unsubstituted, linear or cyclic
v. a short chain alkoxyl, alkenoxyl, or alkynoxyl group, which can be branched or unbranched, substituted or unsubstituted, linear or cyclic; and
vi. a nitrile.

**7.** A compound for use according to Claim 6, wherein $R_{2'}$ is a trifluoromethyl group, and each of $R_{1',}$ and $R_{3'}$-$R_{5'}$ is a hydrogen atom; optionally wherein the compound is sodium 1-amino-9,10-dioxo-4-(3-(trifluoromethyl)phenylamino)-9,10-dihydroanthracene-2-sulfonate.

**8.** A compound for use according to Claim 6, wherein $R_{2'}$ is a trifluoromethyl group, $R_{3'}$ is fluoride, and each of $R_{1'}$, $R_{4'}$, and $R_{5'}$ is a hydrogen atom; optionally wherein the compound is sodium 1-amino-4-(4-fluoro-3-(trifluoromethyl) phenylamino)-9,10-dioxo-9,10-dihydroanthracene-2-sulfonate.

**9.** A compound for use according to Claim 6, wherein $R_{2'}$ is an ethyl group, and each of $R_{1'}$ and $R_{3'}$-$R_{5'}$ is a hydrogen atom; optionally wherein the compound is sodium 1-amino-4-(3-ethylphenylamino)-9,10-dioxo-9,10-dihydroanthracene-2-sulfonate.

**10.** A compound for use according to Claim 6, wherein $R_{2'}$ is a trifluoromethoxyl group ($-OCF_3$), and each of $R_1$, and $R_{3'}$-$R_{5'}$ is a hydrogen atom; optionally wherein the compound is sodium 1 -amino-9,10-dioxo-4-(3-(trifluoromethoxy) phenylamino)-9.10-dihydroanthracene-2-sulfonate.

**11.** A compound for use according to Claim 6, wherein $R_{2'}$is a trifluoromethyl group, $R_{3'}$ is chloride, and each of $R_{1'}$, $R_{4'}$. and $R_{5'}$ is a hydrogen atom; optionally wherein the compound is sodium 1-amino-4-(4-chloro-3-(trifluoromethyl) phenylamino)-9,10-dioxo-9,10-dihydroanthracene-2-sulfonate.

**12.** A compound for use according to Claim 6, wherein $R_{2'}$ is a trifluoromethyl group, $R_{3'}$ is a methyl group, and each of $R_{1'}$, $R_{4'}$, and $R_{5'}$ is a hydrogen atom; optionally wherein the compound is sodium 1-amino-4-(4-methyl-3-(trifluoromethyl)phenylamino)-9,10-dioxo-9,10-dihydroanthracene-2-sulfonate.

**13.** A compound for use according to Claim 6, wherein $R_{3'}$ is a benzyl group, and each of $R_{1'}$, $R_{2'}$, $R_{4'}$, and $R_{5'}$ is a hydrogen atom; optionally wherein the compound is sodium 1-amino-4-(4-benzylphenylamino)-9,10-dioxo-9,10-dihydroanthracene-2-sulfonate.

**14.** A pharmaceutical composition comprising a compound as defined in any one of Claims 1-13.

**15.** A compound as defined in any one of Claims 4-13.

Figure 1

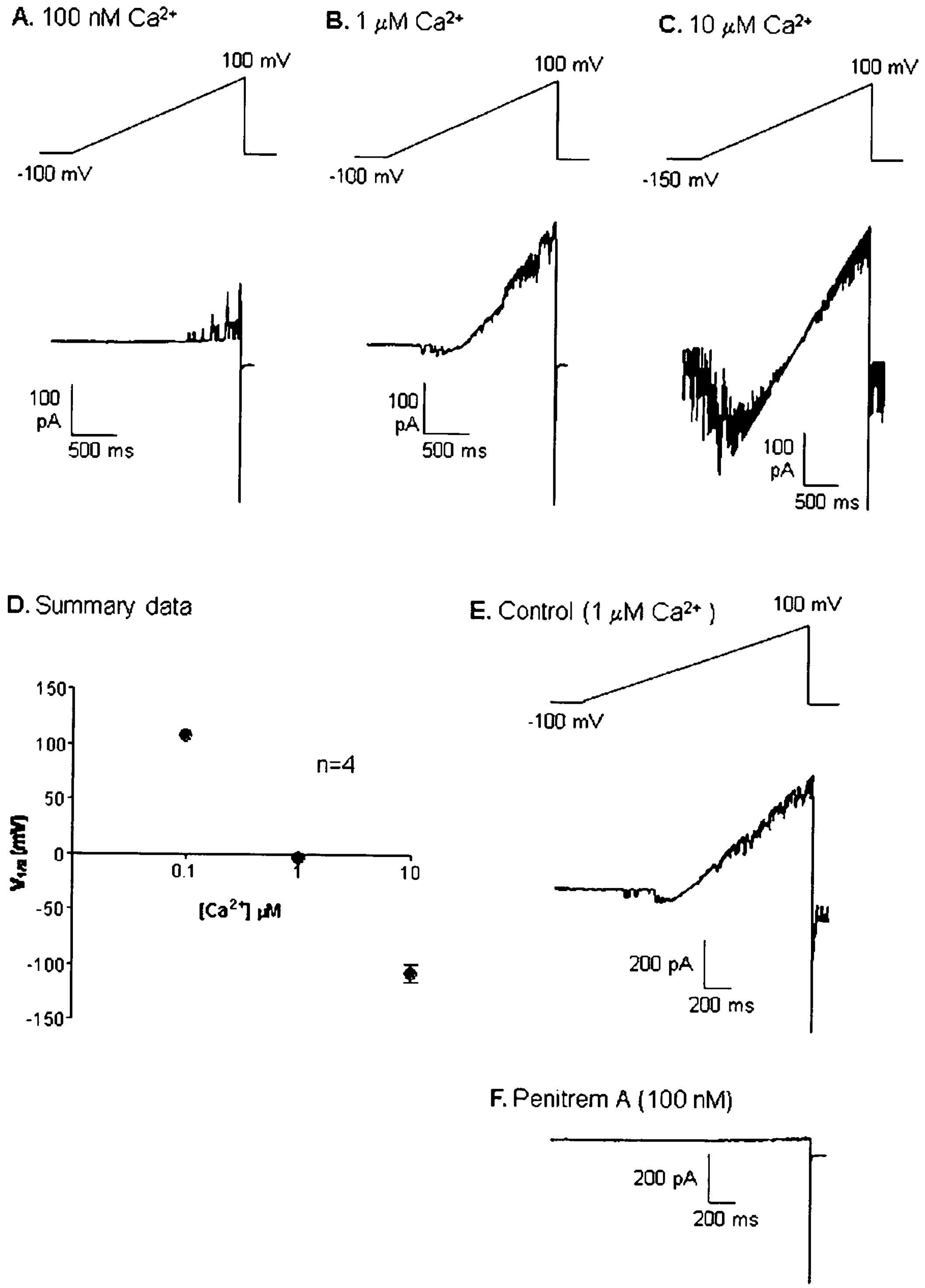
A. 100 nM Ca2+
100 mV
-100 mV
100 pA
500 ms
B. 1 μM Ca2+
100 mV
-100 mV
100 pA
500 ms
C. 10 μM Ca2+
100 mV
-150 mV
100 pA
500 ms
D. Summary data
150
100
50
0
-50
-100
-150
V1/2 (mV)
0.1
1
10
[Ca2+] μM
n=4
E. Control (1 μM Ca2+ )
100 mV
-100 mV
200 pA
200 ms
F. Penitrem A (100 nM)
200 pA
200 ms

Figure 2

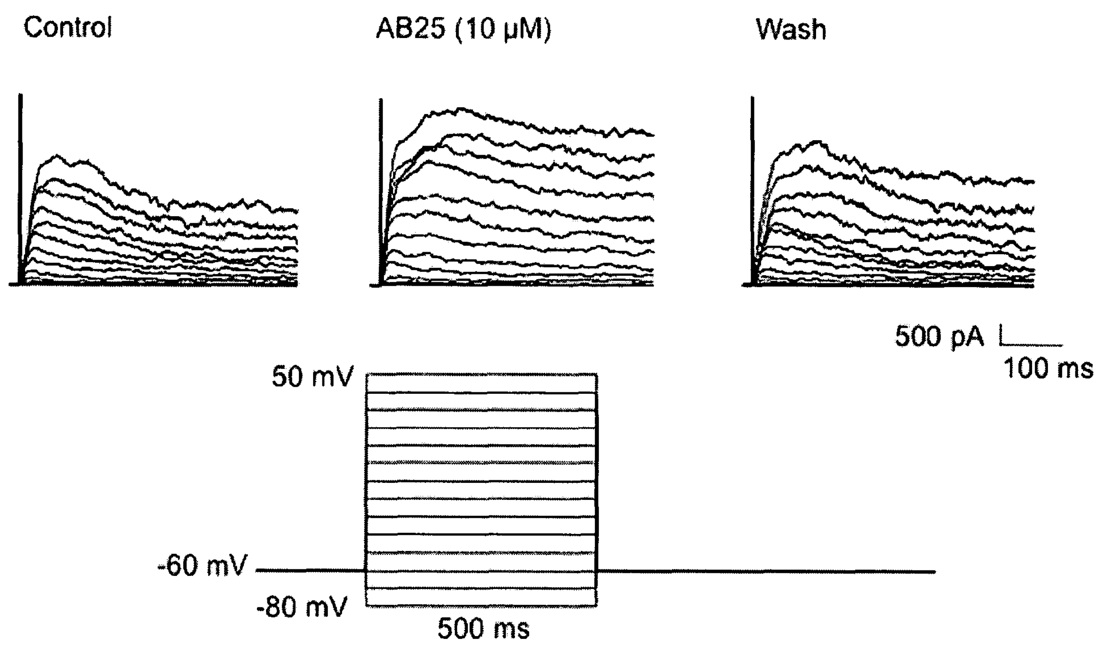


Figure 3

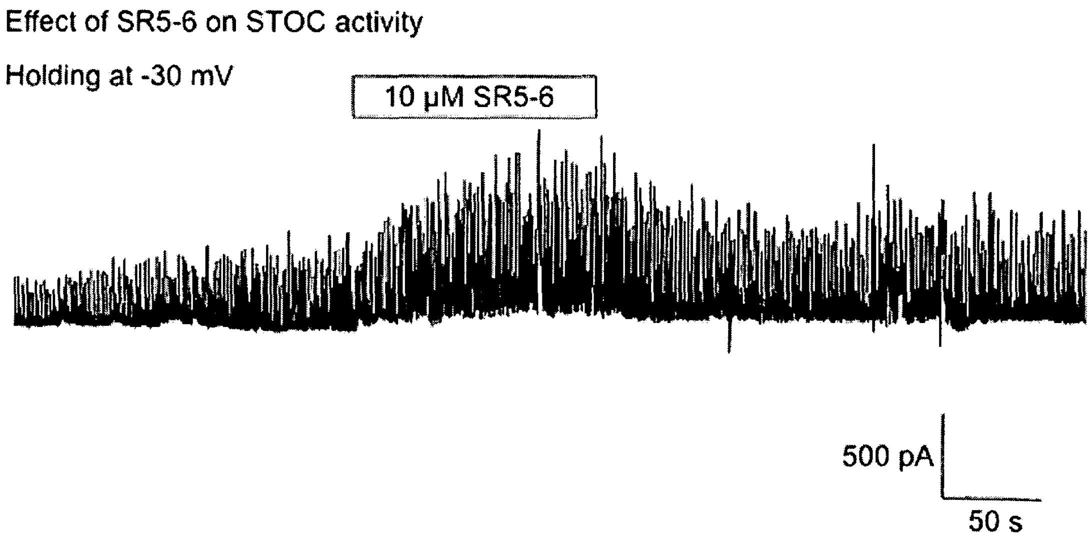

Figure 4

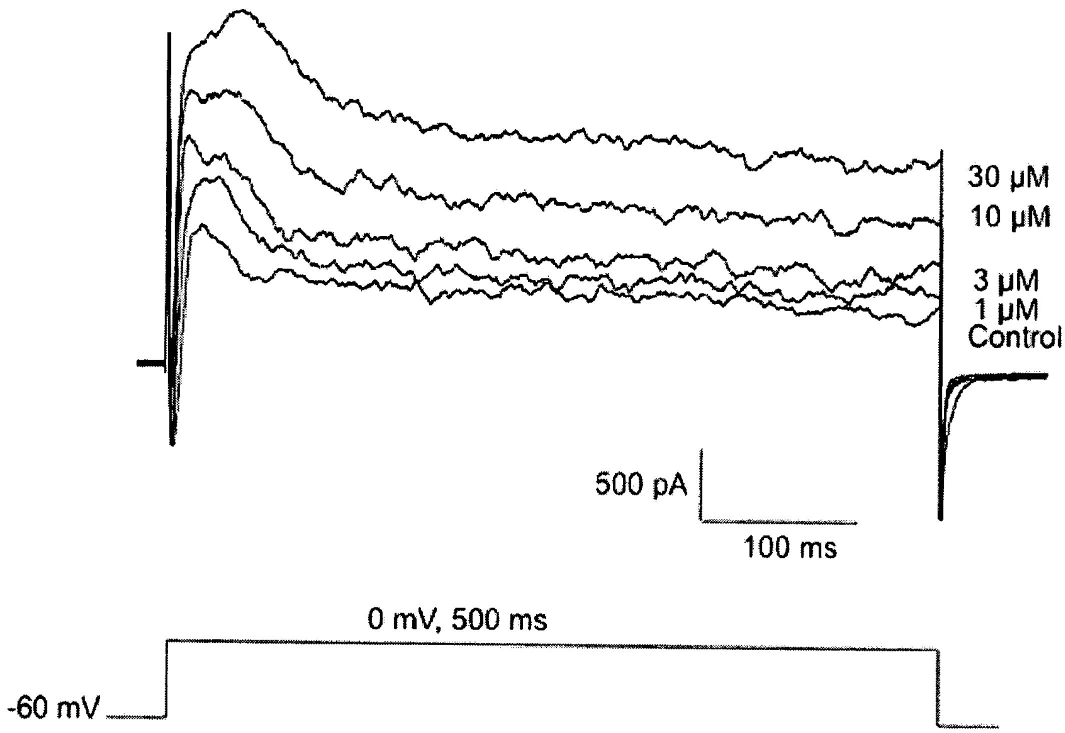


Figure 5

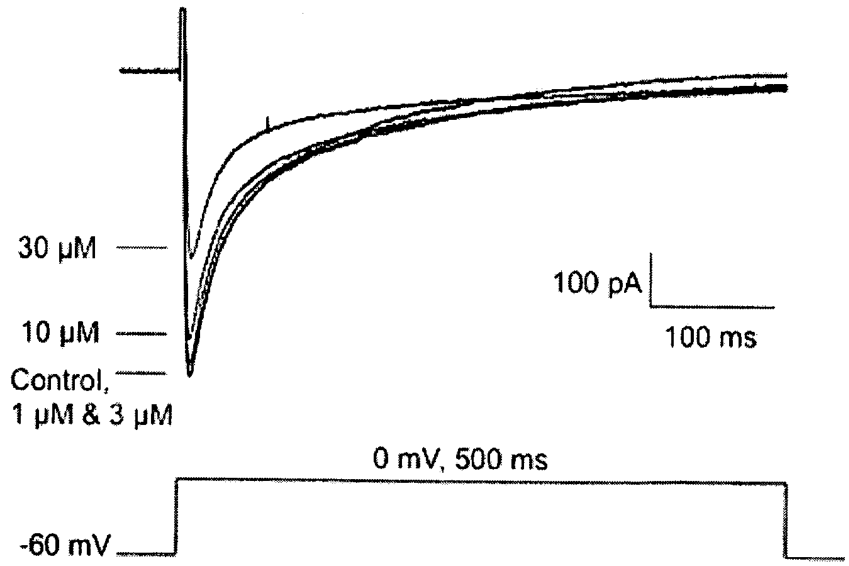

Figure 6

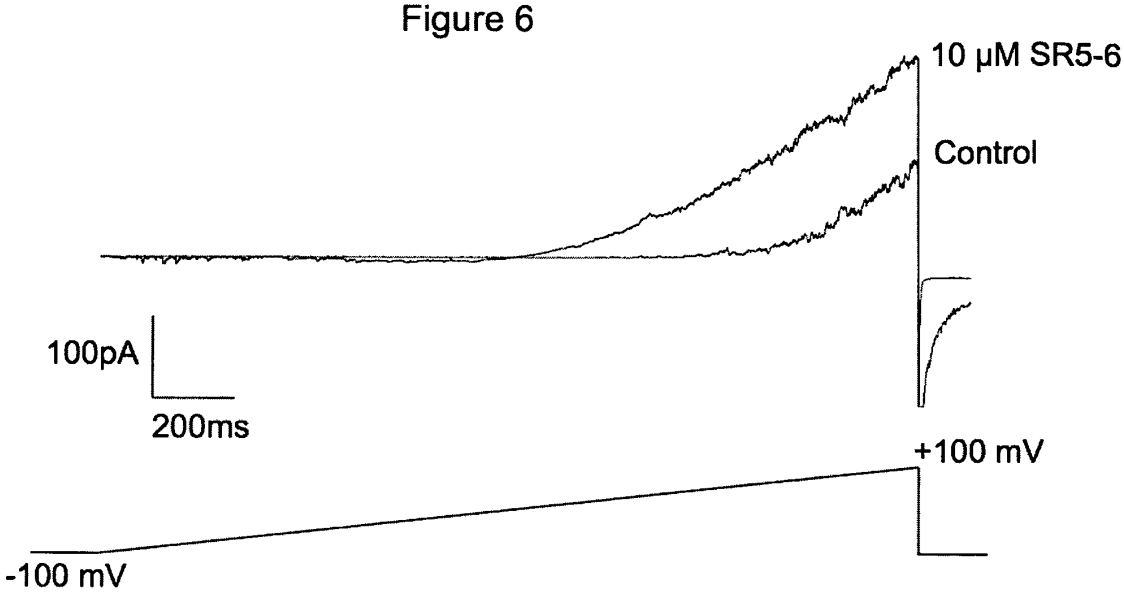

Figure 7

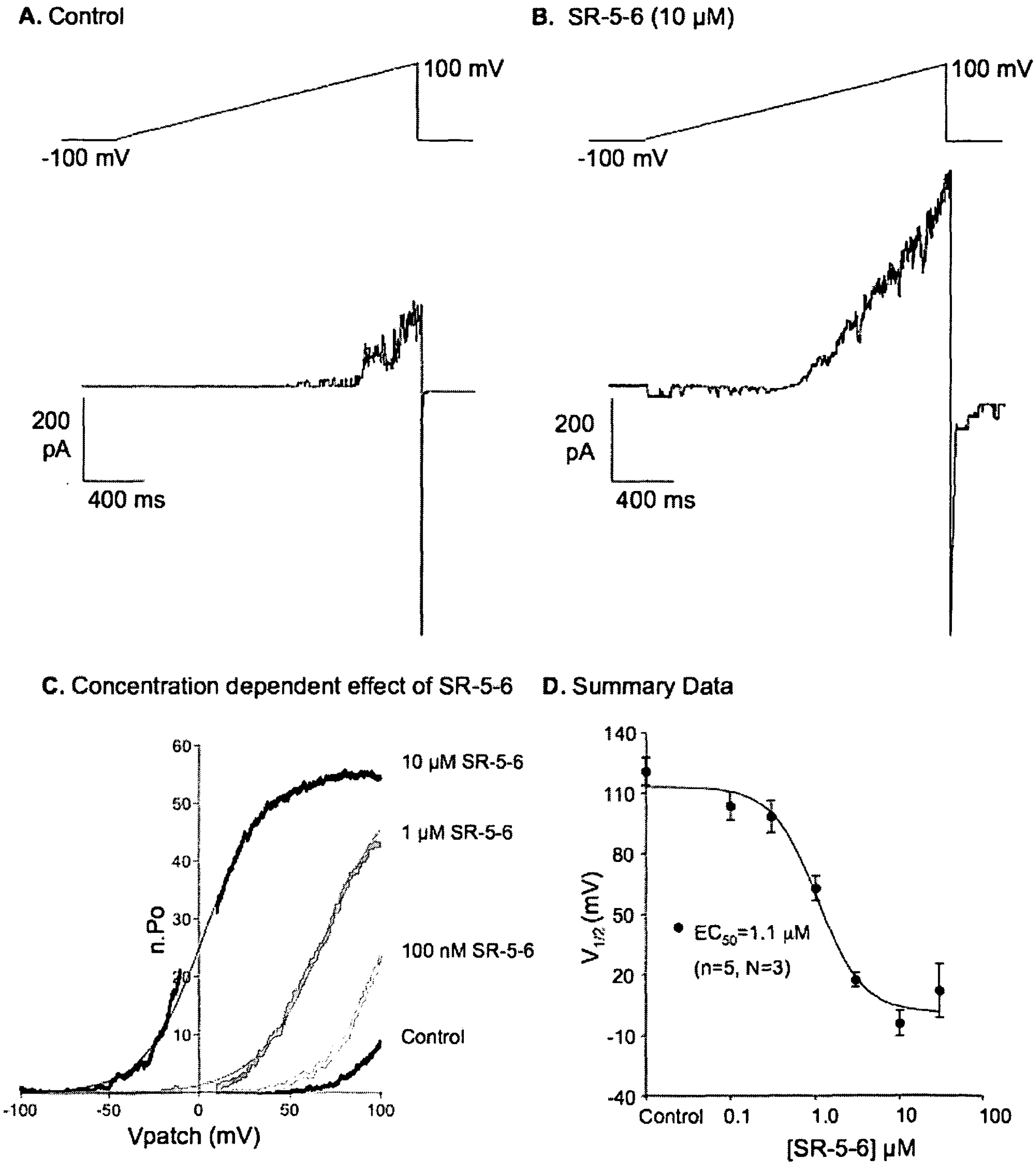
A. Control
B. SR-5-6 (10 μM)
100 mV
-100 mV
100 mV
-100 mV
200
pA
400 ms
200
pA
400 ms
C. Concentration dependent effect of SR-5-6
D. Summary Data
10 μM SR-5-6
1 μM SR-5-6
100 nM SR-5-6
Control
n.Po
Vpatch (mV)
$V_{1/2}$ (mV)
$EC_{50}$=1.1 μM
(n=5, N=3)
[SR-5-6] μM
Control
0.1
1.0
10
100

Europäisches Patentamt
European Patent Office
Office européen des brevets

# EUROPEAN SEARCH REPORT

Application Number
EP 10 00 9835

## DOCUMENTS CONSIDERED TO BE RELEVANT

| | Category | Citation of document with indication, where appropriate, of relevant passages | Relevant to claim | CLASSIFICATION OF THE APPLICATION (IPC) |
|---|---|---|---|---|
| 1 | X | BAQI YOUNIS ET AL: "Development of Potent and Selective Inhibitors of ecto-5 '-Nucleotidase Based on an Anthraquinone Scaffold", JOURNAL OF MEDICINAL CHEMISTRY, vol. 53, no. 5, March 2010 (2010-03), pages 2076-2086, XP002617393, ISSN: 0022-2623<br>* tables 1-2; compounds 12-14, 50, 53 *<br>* page 2076, left-hand column, paragraph 1 - page 2077, left-hand column, paragraph 1 *<br>* page 2077, right-hand column, last paragraph - page 2078, left-hand column, paragraph 1 *<br>----- | 1-5,14, 15 | INV.<br>C07D257/04<br>C07C309/53<br>A61K31/185<br>A61P1/04<br>A61P1/10<br>A61P9/00<br>A61P9/10<br>A61P11/06<br>A61P13/00 |
| 5 | X | DE 456 114 C (IG FARBENINDUSTRIE AG) 15 February 1928 (1928-02-15)<br>* examples 1-2 *<br>----- | 14,15 | |
| 5 | A | US 2004/019042 A1 (LEE CHIH-HUNG [US] ET AL LEE CHIH-HUNG [US] ET AL) 29 January 2004 (2004-01-29)<br>* paragraph [0010] *<br>* examples 10, 13 *<br>* paragraphs [0142] - [0144] *<br>* claim 1 *<br>----- | 1-5,14, 15 | TECHNICAL FIELDS SEARCHED (IPC)<br>A61K<br>C07C<br>C07D |
| 5 | A | JP 2008 214211 A (TOCHIMOTO TENKAIDO CO LTD; HARIMA KANPO SEIYAKU KK) 18 September 2008 (2008-09-18)<br>* paragraphs [0014] - [0018] *<br>* claims 1-2 *<br>----- | 1-5,14, 15 | |
| 5 | A | US 2005/272767 A1 (URADE YOSHIHIRO [JP] ET AL) 8 December 2005 (2005-12-08)<br>* paragraphs [0014] - [0015] *<br>* claims 1-2, 6 *<br>-----<br>-/-- | 1-5,14, 15 | |

1 ~~The present search report has been drawn up for all claims~~

| Place of search | Date of completion of the search | Examiner |
|---|---|---|
| The Hague | 24 January 2011 | Herdemann, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 00 9835

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WEYLER ET AL: "Combinatorial synthesis of anilinoanthraquinone derivatives and evaluation as non-nucleotide-derived P2Y2 receptor antagonists", BIOORGANIC & MEDICINAL CHEMISTRY LETTERS, PERGAMON, ELSEVIER SCIENCE, GB, vol. 18, no. 1, 30 October 2007 (2007-10-30), pages 223-227, XP022410888, ISSN: 0960-894X, DOI: DOI:10.1016/J.CPLETT.2007.12.024 * page 223, left-hand column, paragraph 1 * * figure 1; table 2 * ----- | 1-5,14, 15 | |
| A | BAQI YOUNIS ET AL: "Structure-activity relationships of anthraquinone derivatives derived from bromaminic acid as inhibitors of ectonucleoside triphosphate diphosphohydrolases (E-NTPDases)", PURINERGIC SIGNALLING, vol. 5, no. 1, March 2009 (2009-03), pages 91-106, XP002617392, ISSN: 1573-9538 * page 92, right-hand column, paragraph 3 * * table 1; compounds 11, 13, 20, 21 * * page 98, left-hand column, paragraph 2; figure 4 * ----- -/-- | 1-5,14, 15 | TECHNICAL FIELDS SEARCHED (IPC) |

~~The present search report has been drawn up for all claims~~

| Place of search | Date of completion of the search | Examiner |
|---|---|---|
| The Hague | 24 January 2011 | Herdemann, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 00 9835

## DOCUMENTS CONSIDERED TO BE RELEVANT

| | Category | Citation of document with indication, where appropriate, of relevant passages | Relevant to claim | CLASSIFICATION OF THE APPLICATION (IPC) |
|---|---|---|---|---|
| 7 | A | SUSANNE E HEDE ET AL: "P2Y2 and P2Y4 receptors regulate pancreatic Ca2+-activated K+ channels differently", PFLÜGERS ARCHIV - EUROPEAN JOURNAL OF PHYSIOLOGY, SPRINGER, BERLIN, DE, vol. 450, no. 6, 1 September 2005 (2005-09-01), pages 429-436, XP019344047, ISSN: 1432-2013, DOI: DOI:10.1007/S00424-005-1433-3<br>* the whole document *<br>----- | 1-5,14, 15 | |
| 5 | A | EP 1 634 595 A1 (ROTTAPHARM SPA [IT]) 15 March 2006 (2006-03-15)<br>* paragraphs [0001], [0007] - [0011], [0021] - [0028], [0032] - [0033] *<br>----- | 1-5,14, 15 | |
| | | | | TECHNICAL FIELDS SEARCHED (IPC) |

1 ~~The present search report has been drawn up for all claims~~

| Place of search | Date of completion of the search | Examiner |
|---|---|---|
| The Hague | 24 January 2011 | Herdemann, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 10 00 9835

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

4, 5(completely); 1-3, 14, 15(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION SHEET B**

Application Number
EP 10 00 9835

The Search Division considers that the present European patent application does not comply with the requirements of unity of invention and relates to several inventions or groups of inventions, namely:

1. claims: 4, 5(completely); 1-3, 14, 15(partially)

A compound comprising a substituted anthraquinone and having general formula (IA), or a salt or isomer thereof. A pharmaceutical composition thereof. Said compound for use in treating a disorder caused by or associated with dysfunctional ion channel activity.

---

2. claims: 6-13(completely); 1-3, 14, 15(partially)

A compound comprising a substituted anthraquinone and having general formula (IB), or a salt or isomer thereof. A pharmaceutical composition thereof. Said compound for use in treating a disorder caused by or associated with dysfunctional ion channel activity.

---

3. claims: 1-3, 14(all partially)

A compound comprising a substituted or unsubstituted anthraquinone, or a salt or isomer thereof, as far as neither having general formula (IA) nor general formula (IB) for use in treating a disorder caused by or associated with dysfunctional ion channel activity. A pharmaceutical composition thereof.

---

## ANNEX TO THE EUROPEAN SEARCH REPORT ON EUROPEAN PATENT APPLICATION NO. EP 10 00 9835

This annex lists the patent family members relating to the patent documents cited in the above-mentioned European search report. The members are as contained in the European Patent Office EDP file on
The European Patent Office is in no way liable for these particulars which are merely given for the purpose of information.

24-01-2011

| Patent document cited in search report | Publication date | Patent family member(s) | Publication date |
| --- | --- | --- | --- |
| DE 456114 C | 15-02-1928 | US 1821043 A | 01-09-1931 |
| US 2004019042 A1 | 29-01-2004 | NONE | |
| JP 2008214211 A | 18-09-2008 | NONE | |
| US 2005272767 A1 | 08-12-2005 | JP 4550394 B2 | 22-09-2010 |
| | | JP 2005119984 A | 12-05-2005 |
| EP 1634595 A1 | 15-03-2006 | AT 404195 T | 15-08-2008 |
| | | AU 2005203706 A1 | 09-03-2006 |
| | | CA 2514373 A1 | 19-02-2006 |
| | | DK 1634595 T3 | 24-11-2008 |
| | | ES 2311771 T3 | 16-02-2009 |
| | | JP 2006056890 A | 02-03-2006 |
| | | PT 1634595 E | 28-08-2008 |
| | | SI 1634595 T1 | 31-12-2008 |
| | | US 2006052427 A1 | 09-03-2006 |

EPO FORM P0459

For more details about this annex : see Official Journal of the European Patent Office, No. 12/82